# EUROPEAN PATENT APPLICATION

(11) **EP 4 822 028 A1**
(43) Date of publication of application: **14.10.2026**
(21) Application number: 24901157.8
(22) Date of filing: 09.12.2024
(51) Int. Cl.: C12N 15/86, A61K 39/215, A61K 39/12, A61P 31/14

(54) **ALPHAVIRUS-BASED VECTOR SYSTEM FOR PRODUCING VIRUS-LIKE PARTICLE COMPRISING SELF-AMPLIFYING RNA**

(30) Priority: 07.12.2023 KR 20230177089; 06.12.2024 KR 20240180605
(71) Applicant: Institute for Basic Science, Yuseong-gu Daejeon 34126 (KR)
(72) Inventor: CHOI, Young Ki, Sejong 30098 (KR); KIM, Chul-Jung, Daejeon 34124 (KR); KANG, Yeong-Lim, Daejeon 35211 (KR); YU, Min-Ah, Daejeon 35201 (KR)
(74) Representative: Isarpatent
(86) International application number: PCT/KR2024/020063
(87) International publication number: WO 2025/121971

(57) **Abstract**

One aspect of the present invention provides: a vector including a gene encoding a non-structural alphavirus protein of and a gene encoding a target protein; a cell infected with the vector; a virus-like particle (VLP); and a vaccine composition comprising same. The vector is a single-vector system and can easily and conveniently produce VLP containing saRNA without a helper vector. The VLP and vaccine composition produced through the vector produce a sufficient amount of antibody from only a single administration, have excellent safety due to the absence of additional proliferation, can be administered to mucous membranes, and induces mucous membrane immunity, and thus exhibits a remarkably superior respiratory disease prevention or treatment effect compared to therapeutic agents for intramuscular administration.

## Description

### Technical Field

The present disclosure relates to a vector comprising a gene encoding a nonstructural protein of an alphavirus and a gene encoding a target protein.

### Background Art

A vaccine refers to a pharmaceutical product, such as an attenuated pathogen or a protein, nucleic acid, or the like processed so that the human body can recognize information. Vaccines are generally classified, according to the method of manufacture, into live attenuated vaccines, inactivated vaccines, toxoid vaccines, mRNA vaccines, extract-based vaccines, viral-vector vaccines, virus-like particle (VLP) vaccines, and the like.

Humans are exposed to threats from pathogens through the respiratory tract, and most of these pathogens enter the body through mucosal surfaces. Mucosal immunity, which serves as a first line of defense against such pathogens, may be regarded as one of the most effective ways to block pathogen entry.

A mucosal vaccine can induce a mucosal immune response in mucosal tissue as well as a systemic immune response. In addition, a mucosal vaccine can induce production of antigen-specific IgA at a mucosal surface, which is one of the important features of mucosal immunity. Such vaccines also have advantages in that they do not require an injection needle for vaccination, avoid pain or aversion associated with needle injection, and can be administered safely and easily. Mucosal vaccines based on live attenuated or inactivated vaccine platforms have been commercialized. However, live attenuated vaccines have raised safety concerns, and inactivated vaccines, while safer than live attenuated vaccines, have relatively low immunogenicity and therefore may have difficulty inducing an effective immune response. Accordingly, there remains a need to address issues such as the efficacy and safety of such mucosal vaccines.

An mRNA vaccine induces an immune response by administering mRNA containing viral genetic information, thereby causing antigen to be produced directly in the body. However, in the case of an mRNA vaccine, because only a sequence encoding an antigen protein is included, a sufficient amount of antigen for inducing immunity may not be generated, resulting in a high dosage requirement. In addition, a stable delivery vehicle capable of delivering mRNA into the body is required.

A virus-like particle (VLP) vaccine is a particle formed by specifically expressing viral structural proteins so as to exhibit an outer morphology similar to that of a wild-type virus. Because the VLP vaccine does not contain genetic information of a pathogen inside the particle, it can be used as a delivery vehicle. Further, because the VLP vaccine has a structure similar to the outer morphology of a virus, it can be administered through an infection route of the corresponding virus to induce an immune response.

Accordingly, the present inventors developed a vector system for producing a VLP vaccine containing self-amplifying RNA (saRNA). The VLP vaccine produced using the vector system can be administered through a mucosal route and can induce both mucosal immunity and systemic immunity, demonstrating markedly superior efficacy of the VLP vaccine as a mucosal vaccine for preventing or treating a respiratory disease.

### Disclosure of Invention

### Technical Problem

One aspect provides a vector containing a gene encoding a nonstructural protein of an alphavirus and a gene encoding a target protein.

Another aspect provides a method of preparing a cell for producing virus-like particles (VLPs), the method including introducing RNA expressed from the vector into a cell.

Another aspect provides a cell for producing virus-like particles (VLPs), prepared by the method of preparing a cell for producing VLPs.

Another aspect provides a method of preparing virus-like particles, including culturing the cell for producing virus-like particles.

Another aspect provides virus-like particles prepared by the method of preparing virus-like particles.

Another aspect provides a vaccine composition for preventing or treating a respiratory disease, including the virus-like particles.

Another aspect provides a method of preventing or treating a respiratory disease, including administering a vaccine composition including the virus-like particles to a subject in need thereof.

Another aspect provides use of the virus-like particles for the manufacture of a medicament for preventing or treating a respiratory disease.

### Solution to Problem

One aspect provides a vector including a gene encoding a nonstructural protein of an alphavirus and a gene encoding a target protein.

The term "alphavirus" refers to a small, spherical, enveloped virus that belongs to the family Togaviridae and has a single-stranded, positive-sense RNA genome. Alphaviruses can infect a broad range of cells (e.g., insect, avian, and mammalian cells), and because the viral RNA genome itself encodes an RNA replicase, RNA replication and transcription occur efficiently. The alphavirus genome has two open reading frames (ORFs): a nonstructural ORF and a structural ORF. The first ORF includes the NSP genes and encodes proteins (NSP1 to NSP4) essential for the transcription and translation of viral RNA, producing a single polyprotein precursor, and the polyprotein precursor is cleaved into mature proteins through a proteolytic process. The second ORF encodes three structural proteins: a core nucleocapsid protein C, and envelope proteins P62 and E1.

The term "protein" refers to a molecule in which amino acids are linked via peptide bonds, and the term may be used interchangeably with "polypeptide." The protein includes a recombinant protein, a portion of a protein, or a fragment thereof. For example, it may include a chimeric protein, a peptide or a polypeptide of a size capable of acting as an epitope, and the like.

The term "gene" is to be construed in its broadest sense and may refer to DNA or RNA (specifically mRNA). For example, when the gene is DNA, the gene may be included in a vector in an operable form.

The term "encoding gene" refers to a gene having a nucleotide sequence that encodes the amino acid sequence of a polypeptide or protein. The "encoding gene" includes a gene encoding a portion or fragment of the polypeptide, or a gene encoding a portion or fragment of the protein.

The term "vector" refers to a nucleic acid molecule used to deliver genetic material into a cell in which the genetic material can be replicated and/or expressed. For example, the vector includes a plasmid, a viral vector (bacteriophages, animal viruses, and plant viruses), a cosmid, and an artificial chromosome (e.g., a YAC). Specifically, the vector may be a plasmid, more specifically an alphavirus-derived plasmid, and for example, may be a Semliki Forest virus-derived plasmid.

In an embodiment, the alphavirus may be one or more selected from the group consisting of Aura virus, Babanki virus, Barmah Forest virus, Bebaru virus, Cabassou virus, Chikungunya virus, Eastern equine encephalitis virus, Everglades virus, Fort Morgan virus, Getah virus, Highlands J virus, Kyzylagach virus, Mayaro virus, Me Tri virus, Middelburg virus, Mosso das Pedras virus, Mucambo virus, Ndumu virus, O'nyong-nyong virus, Pixuna virus, Rio Negro virus, Ross River virus, Salmon pancreas disease virus, Semliki Forest virus (SFV), Southern elephant seal virus, Tonate virus, Trocara virus, Una virus, Western equine encephalitis virus, and Whataroa virus, and specifically, may be Semliki Forest virus (SFV).

For example, the nonstructural proteins of the alphavirus may be one or more nonstructural proteins derived from one or more alphaviruses. For example, the nonstructural proteins of the alphavirus may be nonstructural proteins derived from two different alphaviruses, such as a nonstructural protein derived from Aura virus and a nonstructural protein derived from Semliki Forest virus. In addition, the nonstructural proteins of the alphavirus may be nonstructural proteins derived from the same alphavirus. For example, the nonstructural proteins of the alphavirus may be nonstructural proteins derived from Semliki Forest virus.

In an embodiment, the nonstructural proteins of the alphavirus may be one or more selected from the group consisting of an NSP1 protein, an NSP2 protein, an NSP3 protein, and an NSP4 protein. Specifically, the nonstructural proteins of the alphavirus may include all of the NSP1, NSP2, NSP3, and NSP4 proteins of an alphavirus. More specifically, the nonstructural proteins of the alphavirus may include all of the NSP1, NSP2, NSP3, and NSP4 proteins of Semliki Forest virus.

The NSP1 protein has methyltransferase activity and may be involved in viral RNA capping. The NSP2 protein may have helicase and protease activities. The NSP3 protein includes three domains: a macrodomain, a central (or alphavirus-specific) domain, and a hypervariable domain (HVD). The NSP4 protein has RNA-dependent RNA polymerase activity. That is, the NSP4 protein may include a portion or all of an RNA-dependent RNA polymerase, and may include a factor(such as a protein, enzyme, or the like) capable of activating the RNA-dependent RNA polymerase.

In an embodiment, the nonstructural proteins of the alphavirus may include an RNA-dependent RNA polymerase (RdRp). Specifically, the nonstructural proteins of the alphavirus may include the RNA-dependent RNA polymerase (RdRp) of Semliki Forest virus.

The RNA-dependent RNA polymerase is an enzyme that replicates RNA using RNA as a template and is found in archaea and some viruses.

In an embodiment, the gene encoding the nonstructural proteins of the alphavirus may include one or more genes selected from the group consisting of a gene encoding the NSP1 protein of the alphavirus, a gene encoding the NSP2 protein of the alphavirus, a gene encoding the NSP3 protein of the alphavirus, and a gene encoding the NSP4 protein of the alphavirus. Specifically, the gene encoding the nonstructural proteins of the alphavirus may include a gene encoding the NSP1 protein of the alphavirus, a gene encoding the NSP2 protein of the alphavirus, a gene encoding the NSP3 protein of the alphavirus, and a gene encoding the NSP4 protein of the alphavirus. More specifically, the gene encoding the nonstructural proteins of the alphavirus may include a gene encoding the NSP1 protein of Semliki Forest virus, a gene encoding the NSP2 protein of Semliki Forest virus, a gene encoding the NSP3 protein of Semliki Forest virus, and a gene encoding the NSP4 protein of Semliki Forest virus.

In an embodiment, the gene encoding the nonstructural proteins of the alphavirus may include a gene encoding an RNA-dependent RNA polymerase (RdRp). Specifically, the gene encoding the NSP1 protein of the alphavirus, the gene encoding the NSP2 protein of the alphavirus, the gene encoding the NSP3 protein of the alphavirus, and the gene encoding the NSP4 protein of the alphavirus may include a gene encoding an RNA-dependent RNA polymerase (RdRp). More specifically, the gene encoding the NSP1 protein of Semliki Forest virus, the gene encoding the NSP2 protein of Semliki Forest virus, the gene encoding the NSP3 protein of Semliki Forest virus, and the gene encoding the NSP4 protein of Semliki Forest virus may include a gene encoding an RNA-dependent RNA polymerase (RdRp).

In addition, in an embodiment, the vector may include a gene encoding the RNA-dependent RNA polymerase of the alphavirus. Specifically, the vector may include the gene encoding the RNA-dependent RNA polymerase of the alphavirus and a gene encoding the NSP1 protein of the alphavirus, a gene encoding the NSP2 protein of the alphavirus, a gene encoding the NSP3 protein of the alphavirus, and a gene encoding the NSP4 protein of the alphavirus. More specifically, the vector may include a gene encoding the RNA-dependent RNA polymerase of Semliki Forest virus and a gene encoding the NSP1 protein of Semliki Forest virus, a gene encoding the NSP2 protein of Semliki Forest virus, a gene encoding the NSP3 protein of Semliki Forest virus, and a gene encoding the NSP4 protein of Semliki Forest virus.

Specifically, when the vector includes genes encoding the NSP1 to NSP4 proteins among the nonstructural proteins of the alphavirus, expression from the vector also results in expression of all or part of the RNA-dependent RNA polymerase, or of a factor capable of activating the RNA-dependent RNA polymerase, thereby allowing the RNA-dependent RNA polymerase to function normally.

In an embodiment, the gene encoding the NSP1 protein of the alphavirus may be a polynucleotide consisting of a nucleotide sequence having 70 % or more homology to the nucleotide sequence of SEQ ID NO: 1. Specifically, the gene encoding the NSP1 protein of the alphavirus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 1.

In an embodiment, the gene encoding the NSP2 protein of the alphavirus may be a polynucleotide consisting of a nucleotide sequence having 70 % or more homology to the nucleotide sequence of SEQ ID NO: 3. Specifically, the gene encoding the NSP2 protein of the alphavirus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 3.

In an embodiment, the gene encoding the NSP3 protein of the alphavirus may be a polynucleotide consisting of a nucleotide sequence having 70 % or more homology to the nucleotide sequence of SEQ ID NO: 5. Specifically, the gene encoding the NSP3 protein of the alphavirus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 5.

In an embodiment, the gene encoding the NSP4 protein of the alphavirus may be a polynucleotide consisting of a nucleotide sequence having 70 % or more homology to the nucleotide sequence of SEQ ID NO: 7. Specifically, the gene encoding the NSP4 protein of the alphavirus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 7.

In an embodiment, the gene encoding the RNA-dependent RNA polymerase of the alphavirus may be a polynucleotide consisting of a nucleotide sequence having 70 % or more homology to the nucleotide sequence of SEQ ID NO: 9. Specifically, the gene encoding the RNA-dependent RNA polymerase of the alphavirus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 9.

In an embodiment, the vector may further include a regulatory sequence.

The term "regulatory sequence" refers to a nucleic acid having an expression activity that regulates the expression (i.e., transcription and translation) of a target sequence to be expressed or a gene after being operably linked to the target sequence or the gene, and may encompass a promoter, an enhancer, and other expression regulatory elements (e.g., a polyadenylation signal and an element affecting the stability of expressed RNA).

In an embodiment, the regulatory sequence may include one or more selected from the group consisting of a 3'-untranslated region (UTR) sequence, a 5'-UTR sequence, and a poly(A) tail sequence, and specifically, may include all of the 3'-UTR sequence, the 5'-UTR sequence, and the poly(A) tail sequence.

The term "3' untranslated region (3'-UTR)" refers to a region located downstream of the 3' end of an open reading frame and involved in regulating gene expression.

The term "5' untranslated region (5'-UTR)" refers to a region located upstream of the 5' end of an open reading frame and involved in translation initiation.

The term "poly(A) tail" refers to a structure located at the 3' terminal region of RNA that delays exoribonuclease-mediated degradation of the RNA, increases the stability and extends the *in vivo* half-life of the expressed RNA, and thereby affects expression efficiency. The poly(A) tail may generally be formed from a sequence of multiple adenine nucleotides.

In an embodiment, the regulatory sequence may be operably linked.

The term "operably linked" means that an introduced gene is linked to a regulatory sequence in a manner that allows the introduced gene to be expressed in a host cell.

The term "respiratory disease-causing virus" refers to a virus that causes a respiratory disease by infecting mucosal tissue, such as the upper respiratory tract, lower respiratory tract, ocular mucosa, nasal mucosa, oral mucosa, or the like, via droplet transmission or direct contact.

In an embodiment, the target protein may be a protein derived from a target virus or a fragment of the protein. Specifically, the target protein may be a protein derived from a target virus or a fragment of the protein, and the target protein may be one or more selected from the group consisting of a spike protein derived from the target virus or a fragment of the spike protein, an envelope protein derived from the target virus or a fragment of the envelope protein, a membrane protein derived from the target virus or a fragment of the membrane protein, a packaging-signal-related protein derived from the target virus or a fragment of the packaging-signal-related protein, and a nucleocapsid protein derived from the target virus or a fragment of the nucleocapsid protein. More specifically, the target protein may be a protein derived from a target virus or a fragment of the protein, and the protein derived from the target virus or a fragment of the protein may be a combination of a spike protein derived from the target virus or a fragment of the spike protein, an envelope protein derived from the target virus or a fragment of the envelope protein, a membrane protein derived from the target virus or a fragment of the membrane protein, a packaging-signal-related protein derived from the target virus or a fragment of the packaging-signal-related protein, and a nucleocapsid protein derived from the target virus or a fragment of the nucleocapsid protein; or the protein derived from the target virus or a fragment of the protein may be a combination of a spike protein derived from the target virus or a fragment of the spike protein, an envelope protein derived from the target virus or a fragment of the envelope protein, a membrane protein derived from the target virus or a fragment of the membrane protein, and a packaging-signal-related protein derived from the target virus or a fragment of the packaging-signal-related protein.

The term "spike (S) protein" refers to a structural protein that is present on the viral surface and forms club-shaped protrusions. The spike protein is known to bind to a host-cell glycoprotein receptor, and this binding may mediate fusion between the host-cell membrane and the viral envelope. The spike protein may also induce production of neutralizing antibodies.

The term "envelope (E) protein" refers to a protein that forms the outermost layer of a virus and plays a role in protecting the viral genome.

The term "membrane (M) protein" refers to a transmembrane protein that plays a central role in the assembly of viral particles.

The term "nucleocapsid (N) protein" refers to a protein that binds to viral RNA and plays an important role in stabilizing and packaging the viral genome during viral particle assembly, envelope formation, and RNA synthesis.

The term "packaging signal" refers to a signal found in a positive-sense, single-stranded RNA genome, and the packaging signal allows viral structural proteins to assemble into viral particles. The term "packaging-signal-related protein" refers to a protein involved in inducing particle assembly, such as a nonstructural protein, an enzyme, a low-molecular-weight protein, or the like.

In an embodiment, the gene encoding the target protein may be a gene encoding a protein derived from a target virus or a fragment of the protein. Specifically, the gene encoding the target protein may be a gene encoding a protein derived from a target virus or a fragment of the protein, and the gene encoding the target protein may be one or more selected from the group consisting of a gene encoding a spike protein derived from the target virus or a fragment of the spike protein, a gene encoding an envelope protein derived from the target virus or a fragment of the envelope protein, a gene encoding a membrane protein derived from the target virus or a fragment of the membrane protein, a gene encoding a packaging-signal-related protein derived from the target virus or a fragment of the packaging-signal-related protein, and a gene encoding a nucleocapsid protein derived from the target virus or a fragment of the nucleocapsid protein. More specifically, the gene encoding the target protein may be a gene encoding a protein derived from a target virus or a fragment of the protein, and the gene encoding the target protein may be a combination of a gene encoding a spike protein derived from the target virus or a fragment of the spike protein, a gene encoding an envelope protein derived from the target virus or a fragment of the envelope protein, a gene encoding a membrane protein derived from the target virus or a fragment of the membrane protein, a gene encoding a packaging-signal-related protein derived from the target virus or a fragment of the packaging-signal-related protein, and a gene encoding a nucleocapsid protein derived from the target virus or a fragment of the nucleocapsid protein; or a combination of the gene encoding the target protein may be a gene encoding a spike protein derived from the target virus or a fragment of the spike protein, a gene encoding an envelope protein derived from the target virus or a fragment of the envelope protein, a gene encoding a membrane protein derived from the target virus or a fragment of the membrane protein, and a gene encoding a packaging-signal-related protein derived from the target virus or a fragment of the packaging-signal-related protein.

In an embodiment, the target virus may be a respiratory disease-causing virus. Specifically, the protein derived from the target virus or a fragment of the protein may be a protein derived from a respiratory disease-causing virus or a fragment of such a protein. More specifically, the protein derived from the target virus or a fragment of the protein may be one or more selected from the group consisting of a spike protein derived from a respiratory disease-causing virus or a fragment of the spike protein, an envelope protein derived from a respiratory disease-causing virus or a fragment of the envelope protein, a membrane protein derived from a respiratory disease-causing virus or a fragment of the membrane protein, a packaging-signal-related protein derived from a respiratory disease-causing virus or a fragment of the packaging-signal-related protein, and a nucleocapsid protein derived from a respiratory disease-causing virus or a fragment of the nucleocapsid protein. Even more specifically, the protein derived from the target virus or a fragment of the protein may be a combination of a spike protein derived from a respiratory disease-causing virus or a fragment of the spike protein, an envelope protein derived from a respiratory disease-causing virus or a fragment of the envelope protein, a membrane protein derived from a respiratory disease-causing virus or a fragment of the membrane protein, a packaging-signal-related protein derived from a respiratory disease-causing virus or a fragment of the packaging-signal-related protein, and a nucleocapsid protein derived from a respiratory disease-causing virus or a fragment of the nucleocapsid protein; or a combination of the protein derived from the target virus or a fragment of the protein may be a spike protein derived from a respiratory disease-causing virus or a fragment of the spike protein, an envelope protein derived from a respiratory disease-causing virus or a fragment of the envelope protein, a membrane protein derived from a respiratory disease-causing virus or a fragment of the membrane protein, and a packaging-signal-related protein derived from a respiratory disease-causing virus or a fragment of the packaging-signal-related protein.

For example, when the target virus is a coronavirus and the protein derived from the target virus or a fragment of the protein is a spike protein of the coronavirus or a fragment of the spike protein, the protein derived from the target virus or a fragment of the protein refers to the full-length spike protein of the coronavirus, a portion of the spike protein, or a fragment of the spike protein. Specifically, the protein derived from the target virus or a fragment of the protein refers to the full-length spike protein of the coronavirus or a portion thereof, or to a fragment of the spike protein of the coronavirus or a portion of such a fragment.

In addition, the protein derived from the target virus or a fragment of the protein may be prepared by recombining proteins derived from different variant viruses of the same lineage or from viruses of different lineages, or fragments of such proteins. For example, when the protein derived from the target virus or a fragment of the protein is a SARS-CoV-2 spike protein or a fragment of the spike protein, the protein or fragment may refer to a chimeric spike protein or a fragment thereof in which the receptor-binding domain (RBD) portion of the SARS-CoV-2 spike protein, or of a fragment of the SARS-CoV-2 spike protein, is recombined with the receptor-binding domain of MERS-CoV.

In an embodiment, the gene encoding the protein derived from the target virus or a fragment of the protein may be a gene encoding a protein derived from a respiratory disease-causing virus or a fragment of such a protein. Specifically, the gene encoding the protein derived from the target virus or a fragment of the protein may be one or more selected from the group consisting of a gene encoding a spike protein derived from a respiratory disease-causing virus or a fragment of the spike protein, a gene encoding an envelope protein derived from a respiratory disease-causing virus or a fragment of the envelope protein, a gene encoding a membrane protein derived from a respiratory disease-causing virus or a fragment of the membrane protein, a gene encoding a packaging-signal-related protein derived from a respiratory disease-causing virus or a fragment of the packaging-signal-related protein, and a gene encoding a nucleocapsid protein derived from a respiratory disease-causing virus or a fragment of the nucleocapsid protein. More specifically, the gene encoding the protein derived from the target virus or a fragment of the protein may be a combination of a gene encoding a spike protein derived from a respiratory disease-causing virus or a fragment of the spike protein, a gene encoding an envelope protein derived from a respiratory disease-causing virus or a fragment of the envelope protein, a gene encoding a membrane protein derived from a respiratory disease-causing virus or a fragment of the membrane protein, a gene encoding a packaging-signal-related protein derived from a respiratory disease-causing virus or a fragment of the packaging-signal-related protein, and a gene encoding a nucleocapsid protein derived from a respiratory disease-causing virus or a fragment of the nucleocapsid protein; or a combination of the gene encoding the protein derived from the target virus or a fragment of the protein may be a gene encoding a spike protein derived from a respiratory disease-causing virus or a fragment of the spike protein, a gene encoding an envelope protein derived from a respiratory disease-causing virus or a fragment of the envelope protein, a gene encoding a membrane protein derived from a respiratory disease-causing virus or a fragment of the membrane protein, and a gene encoding a packaging-signal-related protein derived from a respiratory disease-causing virus or a fragment of the packaging-signal-related protein.

For example, when the target virus is a coronavirus and the protein derived from the target virus or a fragment of the protein is a spike protein of the coronavirus or a fragment of the spike protein, the gene encoding the protein derived from the target virus or a fragment of the protein refers to a gene encoding the full-length spike protein of the coronavirus, a portion of the spike protein, or a fragment of the spike protein. Specifically, the gene refers to a gene encoding the full-length spike protein of the coronavirus or a portion of the full-length spike protein, or a gene encoding a fragment of the spike protein of the coronavirus or a portion of such a fragment.

In addition, the gene encoding the protein derived from the target virus or a fragment of the protein may be a gene encoding a protein prepared by recombining proteins derived from different variant viruses of the same lineage or from viruses of different lineages, or fragments of such proteins. For example, when the gene encoding the protein derived from the target virus or a fragment of the protein is a gene encoding a spike protein of SARS-CoV-2 virus or a fragment of the spike protein, the gene may refer to a gene encoding a chimeric spike protein or a fragment of the chimeric spike protein in which the receptor-binding domain (RBD) portion of the SARS-CoV-2 spike protein, or of a fragment of the SARS-CoV-2 spike protein, is recombined with the receptor-binding domain of MERS-CoV virus.

In an embodiment, the gene encoding the spike protein derived from the target virus may be a polynucleotide consisting of a nucleotide sequence having 70 % or more homology to the nucleotide sequence of SEQ ID NO: 11, SEQ ID NO: 23, or SEQ ID NO: 25. Specifically, the gene encoding the spike protein derived from the target virus may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 11, SEQ ID NO: 23, or SEQ ID NO: 25.

In an embodiment, the gene encoding the envelope protein may be a polynucleotide consisting of a nucleotide sequence having 70 % or more homology to the nucleotide sequence of SEQ ID NO: 13. Specifically, the gene encoding the envelope protein may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 13.

In an embodiment, the gene encoding the membrane protein may be a polynucleotide consisting of a nucleotide sequence having 70% or more homology to the nucleotide sequence of SEQ ID NO: 15. Specifically, the gene encoding the membrane protein may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 15.

In an embodiment, the gene encoding the nucleocapsid protein may be a polynucleotide consisting of a nucleotide sequence having 70 % or more homology to the nucleotide sequence of SEQ ID NO: 17. Specifically, the gene encoding the nucleocapsid protein may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 17.

In an embodiment, the gene encoding the packaging-signal-related protein may be a polynucleotide consisting of a nucleotide sequence having 70 % or more homology to the nucleotide sequence of SEQ ID NO: 19. Specifically, the gene encoding the packaging-signal-related protein may be a polynucleotide consisting of a nucleotide sequence having 70 % or more, 71 % or more, 72 % or more, 73 % or more, 74 % or more, 75 % or more, 76 % or more, 77 % or more, 78 % or more, 79 % or more, 80 % or more, 81 % or more, 82 % or more, 83 % or more, 84 % or more, 85 % or more, 86 % or more, 87 % or more, 88 % or more, 89 % or more, 90 % or more, 91 % or more, 92 % or more, 93 % or more, 94 % or more, 95 % or more, 96 % or more, 97 % or more, 98 % or more, 99 % or more, or 100 % homology to the nucleotide sequence of SEQ ID NO: 19.

In an embodiment, the respiratory disease-causing virus may be one or more selected from the group consisting of a coronavirus, a respiratory syncytial virus, an adenovirus, an influenza virus, a rhinovirus, an enterovirus, a bocavirus, a parainfluenza virus, a metapneumovirus, an Epstein-Barr virus, a cytomegalovirus, a herpes simplex virus, a measles virus, and a varicella-zoster virus. Specifically, the respiratory disease-causing virus may be one or more selected from the group consisting of a coronavirus, a respiratory syncytial virus, an adenovirus, and an influenza virus, and more specifically, may be a coronavirus.

The protein derived from the target virus or a fragment of the protein may be one or more proteins derived from one or more target viruses or fragments of such proteins. Specifically, the protein derived from the target virus or a fragment of the protein may be one or more proteins derived from one or more respiratory disease-causing viruses or fragments of such proteins. For example, the target protein may be proteins derived from two different viruses (an RSV fusion glycoprotein and SARS-CoV-2 membrane and envelope proteins), proteins derived from two different viral variants (a MERS-CoV spike protein and SARS-CoV-2 membrane and envelope proteins; or a MERS-CoV/SARS-CoV-2 chimeric spike protein and SARS-CoV-2 membrane and envelope proteins), or the like.

In one example, a vector into which a SARS-CoV-2 packaging-signal sequence and sequences encoding the SARS-CoV-2 structural proteins spike (S), envelope (E), and membrane (M) had been inserted in place of the sequence encoding the structural proteins of Semliki Forest virus (SFV), and a vector into which a sequence encoding nucleocapsid had been inserted, were each successfully linearized. When used for cell transfection, the vectors exhibited excellent VLP-producing capability, confirming the stable VLP-producing capability of the vectors (see Example 1).

In addition, when cells of a SARS-CoV-2 nucleocapsid-expressing cell line were transfected with the vector into which a SARS-CoV-2 packaging-signal sequence and sequences encoding the SARS-CoV-2 structural proteins spike (S), envelope (E), and membrane (M) had been inserted, VLPs were also stably produced. These results confirmed that a cell line for large-scale production of the VLPs was also successfully established (see Example 1).

According to one aspect, the vector including a gene encoding alphavirus nonstructural proteins, including NSP1, NSP2, NSP3, NSP4, and RdRp, and a gene encoding target proteins, including a spike protein, an envelope protein, a membrane protein, and a packaging-signal-related protein, is prepared so that the genes may be stably expressed, and therefore achieves a markedly superior VLP production rate. In addition, the vector is a single-vector system capable of producing VLPs without a helper vector, and therefore is convenient to prepare. The VLPs produced using the vector may be administered through a mucosal route, induce mucosal and systemic immunity, and elicit a markedly superior immune response. The VLPs also have excellent safety because they do not reassemble after a single administration.

Another aspect provides a method of preparing a cell for producing virus-like particles (VLPs), the method including introducing RNA expressed from the vector into a cell.

The terms "vector," "gene," "nonstructural protein," "protein," "respiratory disease-causing virus," "structural protein," and the like may have the meanings described above.

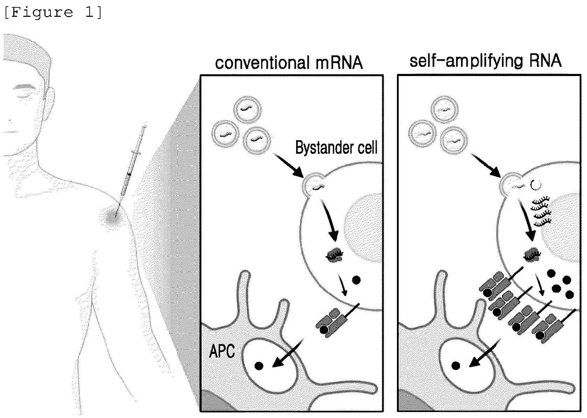

As used herein, the term "RNA" may be used interchangeably with "mRNA" and "saRNA." Specifically, the "RNA" refers to a macromolecule containing a plurality of ribonucleotide units, and more specifically, to a polymer in which a plurality of ribonucleotide units are linked to one another by phosphodiester bonds in a sugar-phosphate backbone. The term "mRNA" refers to RNA that carries genetic information to ribosomes in the cytoplasm. For example, the RNA may be mRNA expressed from the linearized vector. In addition, the "self-amplifying RNA (saRNA)" refers to RNA containing a sequence encoding an RNA-dependent RNA polymerase to enable self-replication within a host cell (FIG. 1). For example, the saRNA may refer to mRNA expressed from a linearized vector including a sequence encoding alphavirus RNA-dependent RNA polymerase.

The term "introduction" refers to delivery of genetic material into a cell and includes transformation, transduction, and transfection. For example, the introduction may be performed via microprojectile bombardment, electroporation, calcium phosphate (CaPO₄) precipitation, calcium chloride (CaCl₂) precipitation, PEG-mediated fusion, microinjection, a liposome-mediated method, or the like.

The term "virus-like particle (VLP)" refers to a particle formed from viral structural proteins having natural self-assembly properties, and VLPs are generally known to mimic the morphology of an actual pathogenic virus and to exhibit highly similar biochemical properties. Specifically, virus-like particles may be prepared to have a structure similar to or identical to that of the original pathogen, and may present antigenic epitopes in a highly repetitive manner.

In an embodiment, introducing the RNA into a cell may include cointroducing another RNA expressed from a vector including: a gene encoding a packaging-signal-related protein derived from a target virus; the polynucleotide derived from the alphavirus; and a gene encoding a second target protein.

The "polynucleotide derived from the alphavirus" refers to a polynucleotide sequence that is not transcribed into RNA or, even if transcribed, is not translated into a protein. For example, the polynucleotide derived from the alphavirus may include a nontranscribed region, an untranslated region, an intergenic region, a regulatory sequence, or the like, and may specifically include a 3'-UTR sequence, a 5'-UTR sequence, a poly(A) tail sequence, an intron, a centromere, a telomere, an origin of replication (Ori), a promoter, an enhancer, a silencer, or the like.

The "second target protein" may be a protein derived from a second target virus or a fragment of such a protein. The second target virus may be the same as or different from the target virus, and the second target protein, i.e., the protein derived from the second target virus or a fragment of such a protein, may be the same as or different from the protein derived from the target virus or a fragment of the protein. For example, when the target virus is a coronavirus, the second target virus may be the same coronavirus as the target virus or may be RSV, which is different from the target virus. When the target protein is a SARS-CoV-2 spike protein or a fragment of the spike protein, the second target protein may be the same as the target protein, i.e., the SARS-CoV-2 spike protein or a fragment of the spike protein, or may be different from the target protein, such as an RSV fusion glycoprotein, a MERS-CoV spike protein, or the like.

In an embodiment, the RNA may include RNA encoding a nonstructural protein of an alphavirus and RNA encoding a target protein. Specifically, the RNA may include RNA encoding an RNA-dependent RNA polymerase of an alphavirus and RNA encoding a target protein.

In an embodiment, the RNA encoding the target protein may be one or more selected from the group consisting of RNA encoding a spike protein derived from a target virus or a fragment of the spike protein, RNA encoding an envelope protein derived from the target virus or a fragment of the envelope protein, RNA encoding a membrane protein derived from the target virus or a fragment of the membrane protein, and RNA encoding a packaging-signal-related protein derived from the target virus or a fragment of the packaging-signal-related protein; and the cell may be genetically engineered to express a nucleocapsid protein derived from the target virus or a fragment of the nucleocapsid protein. Specifically, the RNA may be one or more selected from the group consisting of RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging-signal-related protein derived from the respiratory disease-causing virus; and the cell may be genetically engineered to express the nucleocapsid protein derived from the respiratory disease-causing virus. More specifically, the RNA may be a combination of RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging-signal-related protein derived from the respiratory disease-causing virus.

The term "genetically engineered" encompasses both the act of introducing one or more genetic modifications into a cell and a cell produced by such act. For example, the genetically engineered cell may contain an introduced vector including a gene encoding the nucleocapsid protein of the respiratory disease-causing virus, and may thereby produce or express the nucleocapsid protein of the respiratory disease-causing virus.

In the method according to one aspect, RNA, namely saRNA, expressed from a vector including a gene encoding alphavirus RNA-dependent RNA polymerase (RdRp) and genes encoding target proteins (a spike protein, an envelope protein, a membrane protein, and a packaging-signal-related protein) is introduced into a cell. The saRNA self-amplifies in the cell through the action of RdRp, and the viral structural proteins, namely the spike, envelope, and membrane proteins, are expressed from the saRNA. The packaging signal then enables assembly of the expressed structural proteins into viral particles in which the saRNA is packaged, thereby forming saRNA-containing VLPs. Accordingly, because the saRNA in the VLPs produced in the cells does not include a sequence encoding the viral nucleocapsid, the saRNA-containing VLPs do not give rise to reinfection after a single infection and therefore exhibit markedly high safety.

In an embodiment, the method of preparing a cell for producing virus-like particles may further include linearizing the vector before introducing the RNA into the cell.

In an embodiment, the method of preparing a cell for producing virus-like particles may further include, after linearizing the vector and before introducing the RNA into the cell, transcribing RNA from the linearized vector.

The linearization refers to converting a circular or nicked vector into a form having two ends by cleaving the vector using a physical, chemical, or biological method. Examples of linearizing the vector may include treating the vector with a chemical agent or restriction enzyme, or linearizing the vector by polymerase chain reaction (PCR). When the vector is treated with a restriction enzyme, the restriction enzyme may be SpeI, NheI, BamHI, EcoRI, HindIII, KpnI, NotI, PstI, SmaI, XhoI, FokI, Alw26I, BbvI, BsrI, EarI, HphI, MboI, SfaNI, Tth111I, NeaI, NgoMIV, Eco57I, BcgI, BpI, Bsp24I, BaeI, CjeI, EcoPI, StyLTI, BAL-31, T4, T7, RecBCD, P25736I, P09030II, P1, DNase I, Endo R, Cas9, Cpf1, C2c1, C2c2, C2c3, Cas3, Cas3-HD, Cas5, Cas7, Cas8, Cas10, or the like. Specifically, the restriction enzyme may be SpeI.

In an embodiment, the cell may be a microbial cell, an animal cell, or a plant cell. Specifically, the cell may be a microbial cell, such as a yeast, fungal, or protozoan cell; a plant cell; an insect or amphibian cell; or a mammalian cell, such as a BHK-21, Vero, CHO, HeLa, HEK 293, or COS-1 cell.

In one example, VLPs containing saRNA produced using the vector were found to have an irregular morphology. When the VLPs were produced using a nucleocapsid-expressing cell line, VLPs were produced in an amount sufficient for vaccination. In addition, when 2D and 3D cell lines were infected with the VLPs, expression of SARS-CoV-2 spike protein, which is a constituent protein of the VLPs, was observed in the cells, thereby confirming the infectivity of the VLPs. In addition, the VLPs were not observed in microscopy images 48 hours after inoculation, confirming that the VLPs are a highly safe, non-reinfectious form (see Examples 2 and 3).

In another example, a vector for producing saRNA-containing VLPs was introduced into cells together with a vector for expressing an additional antigen (luciferase). The resulting VLPs packaged not only the saRNA but also RNA encoding the additional antigen, confirming that VLPs capable of co-delivering the RNA encoding the additional antigen could be produced. These results also confirmed that, when the RNA encoding the additional antigen is co-delivered via the VLPs, expression of the additional antigen may be enhanced without interfering with expression from the saRNA encapsulated in the VLPs (see Example 5).

According to one aspect, cells prepared by the method of preparing VLP-producing cells may stably produce VLPs on a large scale. Because the cells themselves express the nucleocapsid protein of the respiratory disease-causing virus, saRNA-containing VLPs produced by the cells do not undergo further propagation after infecting other cells, thereby allowing production of VLPs having markedly superior safety.

Another aspect provides a cell for producing virus-like particles prepared by the method of preparing a cell for producing virus-like particles. Specifically, provided is a cell for producing virus-like particles (VLPs) prepared by a method of preparing a cell for producing virus-like particles, the method including introducing RNA expressed from the vector into a cell.

The terms "vector," "gene," "nonstructural protein," "protein," "respiratory disease-causing virus," "structural protein," "RNA," "introduction," and the like may have the meanings described above.

In an embodiment, the RNA encoding the target protein may be one or more selected from the group consisting of RNA encoding a spike protein derived from a target virus or a fragment of the spike protein, RNA encoding an envelope protein derived from the target virus or a fragment of the envelope protein, RNA encoding a membrane protein derived from the target virus or a fragment of the membrane protein, and RNA encoding a packaging-signal-related protein derived from the target virus or a fragment of the packaging-signal-related protein; and the cell may be genetically engineered to express a nucleocapsid protein derived from the target virus or a fragment of the nucleocapsid protein. Specifically, the RNA may be one or more selected from the group consisting of RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging-signal-related protein derived from the respiratory disease-causing virus; and the cell may be genetically engineered to express the nucleocapsid protein derived from the respiratory disease-causing virus. More specifically, the RNA may be a combination of RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging-signal-related protein derived from the respiratory disease-causing virus.

In an embodiment, the cell may be a microbial cell, an animal cell, or a plant cell. Specifically, the cell may be a microbial cell, such as a yeast, fungal, or protozoan cell; a plant cell; an insect or amphibian cell; or a mammalian cell, such as a BHK-21, Vero, CHO, HeLa, HEK 293, or COS-1 cell.

According to one aspect, cells prepared by the method of preparing VLP-producing cells may stably produce VLPs on a large scale, that is, may efficiently produce VLPs in an amount sufficient for vaccine inoculation. Because the cells themselves express the viral nucleocapsid protein, the saRNA expressed from the vector does not include a sequence encoding the viral nucleocapsid. Accordingly, saRNA-containing VLPs produced by the cells do not undergo further propagation after infecting other cells, thereby allowing production of VLPs having markedly superior safety.

Another aspect provides a method of preparing virus-like particles, the method including culturing the cell for producing virus-like particles. Specifically, provided is a method of preparing virus-like particles (VLPs), the method including culturing the cell for producing virus-like particles, the cell being prepared by a method of preparing a cell for producing virus-like particles, the method including introducing RNA expressed from the vector into a cell.

The terms "vector," "gene," "nonstructural protein," "protein," "respiratory disease-causing virus," "structural protein," "RNA," "introduction," and the like may have the meanings described above.

In the method according to one aspect, upon culturing the cell for producing virus-like particles, the RNA expressed from the vector is replicated by RNA-dependent RNA polymerase, and the proteins encoded by the RNA are expressed. Accordingly, VLPs in which the RNA is packaged are produced in large quantities.

In an embodiment, the method of preparing virus-like particles may further include disrupting the cell membrane of the cell.

Specifically, in the method of preparing virus-like particles, disrupting the cell membrane includes lysing the cell to recover virus-like particles produced within the cell. Specifically, disrupting the cell membrane may be performed by a physical, chemical, or biological method, such as sonication, disruption by agitation, homogenization, repeated freeze-thaw cycles, osmotic shock, use of a lytic enzyme, use of an organic solvent, use of a surfactant, or a combination thereof.

When the method of preparing virus-like particles further includes disrupting the cell membrane, according to the method of preparing VLPs, VLPs may be produced at a markedly high yield.

In an embodiment, the method of preparing virus-like particles may further include purifying the virus-like particles after disrupting the cell membrane of the cell.

Specifically, "purification" refers to a method of removing impurities present with the produced virus-like particles and increasing the purity so that only the virus-like particles are present. For example, purifying the virus-like particles may be performed by filtration, ultrafiltration, centrifugation, chromatography, or a combination thereof.

Specifically, when the method of preparing virus-like particles further includes purifying the virus-like particles, VLPs may be produced with markedly high purity.

Since virus-like particles (VLPs) prepared according to one aspect contain saRNA internally and have an external structure corresponding to that of a respiratory disease-causing virus, the VLPs may be administered through a mucosal route, which is a route of infection for the respiratory disease-causing virus. In addition, the saRNA contained therein may self-amplify and drive production of an amount of antigen sufficient to elicit immunity after a single administration. The VLPs induce mucosal and systemic immunity when administered through a mucosal route, thereby eliciting a markedly superior immune response compared with intramuscular administration. In addition, because the saRNA does not include a sequence encoding nucleocapsid, the VLPs do not undergo further propagation and therefore also exhibit markedly superior safety.

Accordingly, the prepared virus-like particles (VLPs) may be administered through a mucosal route and, after administration, may induce mucosal and systemic immune responses with a single administration, without further propagation, thereby rapidly inducing antibody production. Thus, when the VLPs are used in a vaccine, a vaccine having markedly superior immunogenicity and safety compared with an intramuscularly administered vaccine may be prepared.

Another aspect provides virus-like particles prepared by the method of preparing virus-like particles. Specifically, the method of preparing virus-like particles may include culturing the cell for producing virus-like particles, the cell being prepared by a method of preparing a cell for producing virus-like particles, the method including introducing RNA expressed from the vector into a cell. The vector may include a gene encoding a nonstructural protein of an alphavirus and a gene encoding a target protein.

The terms "vector," "gene," "nonstructural protein," "protein," "respiratory disease-causing virus," "structural protein," "RNA," "introduction," "virus-like particle," and the like may have the meanings described above.

In an embodiment, the target protein may be a protein derived from a target virus or a fragment of the protein. Specifically, the target protein may include one or more selected from the group consisting of a spike protein derived from the target virus or a fragment of the spike protein, an envelope protein derived from the target virus or a fragment of the envelope protein, and a membrane protein derived from the target virus or a fragment of the membrane protein. More specifically, the target protein may be a combination of a spike protein derived from the target virus or a fragment of the spike protein, an envelope protein derived from the target virus or a fragment of the envelope protein, and a membrane protein derived from the target virus or a fragment of the membrane protein. Even more specifically, the target protein may be a combination of a spike protein derived from a respiratory disease-causing virus or a fragment of the spike protein, an envelope protein derived from a respiratory disease-causing virus or a fragment of the envelope protein, and a membrane protein derived from a respiratory disease-causing virus or a fragment of the membrane protein.

The protein derived from the target virus or a fragment of the protein may be proteins derived from one or more target viruses or fragments of such proteins. Specifically, the protein derived from the target virus or a fragment of the protein may be proteins derived from one or more respiratory disease-causing viruses or fragments of such proteins. For example, the target protein may be proteins derived from two different viruses (an RSV fusion glycoprotein and SARS-CoV-2 membrane and envelope proteins); proteins derived from two different viral variants (a MERS-CoV spike protein and SARS-CoV-2 membrane and envelope proteins; or a MERS-CoV/SARS-CoV-2 chimeric spike protein and SARS-CoV-2 membrane and envelope proteins); or the like.

In an embodiment, the RNA may be packaged with the spike protein derived from the target virus or a fragment of the spike protein, the envelope protein derived from the target virus or a fragment of the envelope protein, and the membrane protein derived from the target virus or a fragment of the membrane protein.

In an embodiment, the RNA may include RNA encoding a nonstructural protein of an alphavirus and RNA encoding a target protein. Specifically, the RNA may include RNA encoding an RNA-dependent RNA polymerase of an alphavirus and RNA encoding a target protein.

In an embodiment, the RNA encoding the target protein may be one or more selected from the group consisting of RNA encoding a spike protein derived from a target virus or a fragment of the spike protein, RNA encoding an envelope protein derived from the target virus or a fragment of the envelope protein, RNA encoding a membrane protein derived from the target virus or a fragment of the membrane protein, and RNA encoding a packaging-signal-related protein derived from the target virus or a fragment of the packaging-signal-related protein. Specifically, the RNA may be one or more selected from the group consisting of RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging-signal-related protein derived from the respiratory disease-causing virus. More specifically, the RNA may be RNA encoding a spike protein derived from a respiratory disease-causing virus, RNA encoding an envelope protein derived from the respiratory disease-causing virus, RNA encoding a membrane protein derived from the respiratory disease-causing virus, and RNA encoding a packaging signal-related protein derived from the respiratory disease-causing virus.

In an embodiment, the virus-like particles may further contain RNA expressed from a vector including: a gene encoding a packaging-signal-related protein derived from a target virus; a polynucleotide derived from the alphavirus; and a gene encoding a second target protein.

The "polynucleotide derived from the alphavirus" refers to a polynucleotide sequence that is not transcribed into RNA or, even if transcribed, is not translated into a protein. For example, the polynucleotide derived from the alphavirus may include a nontranscribed region, an untranslated region, an intergenic region, a regulatory sequence, or the like, and may specifically include a 3'-UTR sequence, a 5'-UTR sequence, a poly(A) tail sequence, an intron, a centromere, a telomere, an origin of replication (Ori), a promoter, an enhancer, a silencer, or the like.

The "second target protein" may be a protein derived from a second target virus or a fragment of such a protein. The second target virus may be the same as or different from the target virus, and the second target protein, i.e., the protein derived from the second target virus or a fragment of such a protein, may be the same as or different from the protein derived from the target virus or a fragment of the protein. For example, when the target virus is a coronavirus, the second target virus may be the same coronavirus as the target virus or may be RSV, which is different from the target virus. When the target protein is a SARS-CoV-2 spike protein or a fragment of the spike protein, the second target protein may be the same as the target protein, i.e., the SARS-CoV-2 spike protein or a fragment of the spike protein, or may be different from the target protein, such as an RSV fusion glycoprotein, a MERS-CoV spike protein, or the like.

The virus-like particles may be for intraperitoneal, intramucosal, intramuscular, intradermal, subcutaneous, intravenous, intrarectal, intrapulmonary, intrathecal, oral, transdermal, or intranasal administration, and specifically, may be for intramucosal or intranasal administration.

In an embodiment, the virus-like particles may induce a mucosal immune response.

The term "mucosa" refers to epithelial cells in the body having mucous glands among skin tissues, and is a tissue forming the internal linings of various body cavities. For example, the mucosa may be a nasal mucosa, a nasal cavity mucosa, an oral mucosa, an ocular mucosa, an otic mucosa, a genital mucosa, a throat mucosa, a pharyngeal mucosa, an airway mucosa, a bronchial mucosa, a pulmonary mucosa, a gastric mucosa, an intestinal mucosa, a rectal mucosa, or the like.

The term "mucosal immune response" refers to an immune response induced in a mucosa. For example, the mucosal immune response includes antigen-specific immunoglobulin G (IgG) and subclasses thereof, immunoglobulin A (IgA) and subclasses thereof, immunoglobulin M (IgM) and subclasses thereof, and cell-mediated immunity against the immunizing antigen.

In an embodiment, the virus-like particles may induce one or more selected from the group consisting of a cellular immune response and a humoral immune response at a mucosal surface, and specifically, may induce both a cellular immune response and a humoral immune response.

In an embodiment, the virus-like particles may induce one or more selected from the group consisting of a local immune response and a systemic immune response, and specifically, may induce both a local immune response and a systemic immune response.

The virus-like particles according to one aspect, formulated as a vaccine for intramucosal or intranasal administration, not only induce a mucosal immune response that produces antigen-specific IgA at a mucosal surface but also simultaneously induce a systemic immune response, thereby eliciting a markedly enhanced immune response compared to conventional intramuscular vaccines and rapidly inducing an immune response. In addition, since vaccination is not performed via an injection needle, it avoids pain or aversion, allowing for safe and easy administration. Specifically, intranasal administration of the virus-like particles may elicit a rapid and enhanced immune response against a virus that infects a host through the mucosal surfaces of the upper and/or lower respiratory tracts.

In an embodiment, the mucosal immune response may involve the production of one or more selected from the group consisting of antigen-specific IgA and IgG in a mucosa; specifically, may involve the production of antigen-specific IgA and IgG in a mucosa; and more specifically, may involve the production of antigen-specific IgA in a mucosa.

When the virus-like particles are administered via a mucosa, which is a primary infection route of the respiratory disease-causing virus, antigen-specific IgA is produced through the mucosal immune response, and systemic immunity is rapidly established. Consequently, they elicit a markedly enhanced immune response compared to administration via intramuscular injection or the like, thereby increasing the vaccination efficiency of a vaccine including the virus-like particles.

Since virus-like particles prepared according to one aspect contain saRNA internally and have an external structure corresponding to that of a respiratory disease-causing virus, the VLPs may be administered through a mucosal route, which is a route of infection for the respiratory disease-causing virus. In addition, the saRNA contained therein may self-amplify and drive production of an amount of antigen sufficient to elicit immunity after a single administration. In addition, because the saRNA does not include a sequence encoding nucleocapsid, the VLPs do not undergo further propagation and therefore also exhibit markedly superior safety.

Accordingly, the prepared virus-like particles (VLPs) may be administered through a mucosal route and, after administration, may induce mucosal and systemic immune responses with a single administration, without further propagation, thereby rapidly inducing antibody production. Thus, when the VLPs are used in a vaccine, a vaccine having markedly superior immunogenicity and safety compared with an intramuscularly administered vaccine may be prepared.

Another aspect provides a vaccine composition for preventing or treating a respiratory disease, the composition including the virus-like particles.

The terms "vector," "gene," "nonstructural protein," "protein," "respiratory disease-causing virus," "structural protein," "RNA," "introduction," "virus-like particle," "mucosa," "mucosal immune response," and the like may have the meanings described above.

The term "vaccine" refers to a biological preparation including an antigen that induces immunity *in vivo*, and refers to an immunogen or an antigenic substance administered to a subject to produce immunity *in vivo* for the prevention or treatment of an infectious disease.

The term "prevention" may refer to any act of suppressing or delaying a respiratory disease in a subject by the administration of the vaccine composition according to one aspect.

The term "treatment" may refer to any act of ameliorating or beneficially altering the symptoms of a respiratory disease in a subject by the administration of the vaccine composition according to one aspect.

The vaccine composition may include one or more immunologically acceptable carriers, excipients, or diluents.

Specifically, the carrier, excipient, or diluent may be, for example, glucose, lactose, dextrose, sucrose, sorbitol, mannitol, xylitol, maltitol, starch, glycerin, starch, acacia gum, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, mineral oil, or the like.

In an embodiment, the vaccine composition may further include an adjuvant. The adjuvant may be aluminum hydroxide (Al(OH)₃), aluminum phosphate (AlPO₄), alum (potassium aluminum sulfate), MF59, virosomes, AS04 [a mixture of aluminum hydroxide and monophosphoryl lipid A (MPL)], AS03 (a mixture of DL-α-tocopherol, squalene, and polysorbate 80, which is an emulsifier), CpG, flagellin, Poly(I:C), AS01, AS02, ISCOMs, ISCOMATRIX, or the like.

In an embodiment, the adjuvant may be contained in the vaccine composition at 10 to 70 v/v%, specifically 30 to 70 v/v%, and more specifically 50 to 70 v/v%. For example, the adjuvant may be contained in the vaccine composition at 50 to 70 v/v%, 50 to 65 v/v%, 50 to 60 v/v%, 50 to 55 v/v%, 55 to 70 v/v%, 55 to 65 v/v%, 55 to 60 v/v%, 60 to 70 v/v%, 60 to 65 v/v%, or 65 to 70 v/v%.

The vaccine composition may be for intraperitoneal, intramucosal, intramuscular, intradermal, subcutaneous, intravenous, intrarectal, intrapulmonary, intrathecal, oral, transdermal, or intranasal administration, and specifically, may be formulated for intramucosal or intranasal administration.

The vaccine composition according to one aspect may be a vaccine suitable for intramucosal or intranasal administration and may induce not only a mucosal immune response that produces antigen-specific IgA at a mucosal surface, but also a systemic immune response, thereby eliciting a markedly superior immune response compared with conventional intramuscularly administered vaccines and inducing a rapid immune response. In addition, because vaccination does not require an injection needle, pain or aversion associated with needle injection can be avoided, and the vaccine composition may be administered safely and easily. Specifically, when the vaccine composition is administered intranasally, the vaccine composition may elicit a rapid and enhanced immune response against a virus that infects a host through the mucosal surfaces of the upper and/or lower respiratory tract.

The vaccine composition may be formulated for use as an oral formulation, such as a powder, granule, tablet, capsule, suspension, emulsion, syrup, or aerosol; an intranasal formulation, such as drops or a spray; or a sterile injectable solution. When formulated, the vaccine composition may be prepared using commonly used diluents or excipients, such as fillers, bulking agents, binders, wetting agents, disintegrants, and surfactants.

Solid preparations for oral administration include tablets, pills, powders, granules, capsules, and the like. Such solid preparations may be prepared by mixing the composition with at least one excipient, such as lecithin-like emulsifier, starch, calcium carbonate, glucose, sucrose, lactose, gelatin, or the like. In addition to simple excipients, lubricants such as magnesium stearate and talc may also be used. Liquid preparations for oral administration may include suspensions, oral solutions, emulsions, syrups, or the like. In addition to commonly used simple diluents such as water and liquid paraffin, the liquid preparations may include various excipients, such as wetting agents, sweeteners, flavoring agents, and preservatives. Preparations for parenteral administration include sterile aqueous solutions, non-aqueous preparations, suspensions, emulsions, and lyophilized preparations. For non-aqueous preparations and suspensions, propylene glycol, polyethylene glycol, vegetable oils such as olive oil, injectable esters such as ethyl oleate, or the like may be used. Penetration enhancers suitable for preparations for intranasal administration are generally known to those skilled in the art, and suitable formulations may be formulated to be sterile, isotonic, and buffered for stability and patient compliance. Preparations for intranasal administration may also be formulated to stimulate mucous secretions in various respects so as to maintain normal ciliary function. The preparations may be maintained at a pH of 5.5 to 6.5 and may include antimicrobial preservatives and suitable stabilizers.

In an embodiment, the respiratory disease may be one or more viral infections selected from the group consisting of a coronavirus infection, a human respiratory syncytial virus infection, an adenovirus infection, an influenza virus infection, a rhinovirus infection, an enterovirus infection, a bocavirus infection, a parainfluenza virus infection, a metapneumovirus infection, an Epstein-Barr virus infection, a cytomegalovirus infection, a herpes simplex virus infection, a measles virus infection, and a varicella-zoster virus infection.

The vaccine composition may be administered in an immunologically effective amount. The term "immunologically effective amount" refers to an amount sufficient to treat a disease with a reasonable benefit/risk ratio applicable to medical treatment. The effective dose level may be determined based on factors including the type and severity of the patient's disease, the activity of the drug, the patient's sensitivity to the drug, the time of administration, the route of administration, the excretion rate, the duration of treatment, concomitantly used drugs, and other factors well known in the medical field. The administration may be carried out once a day or may be divided in multiple doses. For example, the vaccine composition may be administered 1, 2, 3, 4, 5, 6, 7, 8, 9, 10, or more times, and the doses may be administered at intervals of about 1 to 16 weeks, about 1 to 12 weeks, about 1 to 8 weeks, or about 1 to 4 weeks. In the event of a secondary or subsequent infection with a virus targeted by the vaccine composition, the vaccine composition may be re-administered periodically.

The term "administration" refers to delivering a predetermined substance to a subject by an appropriate method. The term "subject" refers to any organism capable of having or developing a respiratory disease, including a human, rat, mouse, pig, horse, cow, livestock, or the like. As a specific example, the subject may be a mammal including a human.

In an embodiment, a single dose of the vaccine composition may have a volume of 0.1 µL to 0.6 mL, specifically 10 µL to 0.6 mL, and more specifically 30 µL to 0.6 mL.

The vaccine composition may include the virus-like particles in an amount of 5 µg to 5000 µg, 5 µg to 2500 µg, 5 µg to 1000 µg, 5 µg to 500 µg, 5 µg to 250 µg, 5 µg to 100 µg, 5 µg to 50 µg, 50 µg to 5000 µg, 50 µg to 2500 µg, 50 µg to 1000 µg, 50 µg to 500 µg, 50 µg to 250 µg, 50 µg to 100 µg, 100 µg to 5000 µg, 100 µg to 2500 µg, 100 µg to 1000 µg, 100 µg to 500 µg, 100 µg to 250 µg, 250 µg to 5000 µg, 250 µg to 2500 µg, 250 µg to 1000 µg, 250 µg to 500 µg, 500 µg to 5000 µg, 500 µg to 2500 µg, 500 µg to 1000 µg, 1000 µg to 5000 µg, 1000 µg to 2500 µg, or 2500 µg to 5000 µg per single dose of the vaccine composition.

The vaccine composition may be administered as a booster vaccine. The term "booster" refers to a second antigenic stimulus administered after a first antigenic stimulus during vaccination. When antibodies are produced, the second antigenic stimulus induces antibody production more rapidly and at a higher rate than the response following the first antigenic stimulus. The "booster vaccine" is administered after a first or second dose of the vaccine composition and may correspond to, for example, a third dose. Broadly, the term refers to any dose administered after the first dose that can provide an immune-enhancing effect.

In an embodiment, the respiratory disease may be one or more selected from the group consisting of an inflammatory respiratory lung disease, chronic obstructive pulmonary disease (COPD), sinusitis, allergic rhinitis, a lower respiratory tract infection, tracheitis, acute bronchitis, chronic bronchitis, emphysema, pneumonia, asthma, bronchiectasis, emphysema, pulmonary tuberculosis, acute respiratory distress syndrome, a diffuse interstitial lung disease, cystic fibrosis, otitis media, bronchiolitis, and pulmonary fibrosis.

In an embodiment, the vaccine composition may induce a mucosal immune response.

In an embodiment, the vaccine composition may induce, at a mucosal surface, one or more immune responses selected from the group consisting of a cellular immune response and a humoral immune response, and specifically, may induce both a cellular immune response and a humoral immune response.

In an embodiment, the vaccine composition may induce one or more immune responses selected from the group consisting of a local immune response and a systemic immune response, and specifically, may induce both a local immune response and a systemic immune response.

In an embodiment, the mucosal immune response may include production, in a mucosa, of one or more selected from the group consisting of antigen-specific IgA and antigen-specific IgG; specifically, may include production, in a mucosa, of antigen-specific IgA and antigen-specific IgG; and more specifically, may include production, in a mucosa, of antigen-specific IgA.

When the vaccine composition is administered via the mucosa, the primary infection route of the respiratory disease-causing virus, it induces the production of antigen-specific IgA through a mucosal immune response and rapidly establishes systemic immunity, thereby eliciting a markedly enhanced immune response compared to administration via intramuscular injection or the like and increasing vaccination efficiency.

In one example, when the VLPs containing the saRNA were intranasally administered to mice, the titers of IgG and the mucosal immune antibody IgA were markedly elevated. Furthermore, the neutralizing antibody titer, the secretion of INF-γ, the expression of INF-α and INF-β genes, and the number of CD4⁺ T cells were significantly greater than those in the control group, the intramuscular administration group, and the group receiving intranasal administration of LNPs.

In addition, when mice were challenged with the virus after vaccination, the group receiving intranasal administration of the VLPs containing the saRNA exhibited recovery of body weight, survival rate, viral titers in the lungs and mucosa, and lung function to normal ranges. These recoveries were significantly superior to those in the intramuscular administration group and the group receiving intranasal administration of LNPs. These results demonstrated that intranasal administration of the VLPs provides robust immunogenicity and protective efficacy against viral challenge (see Example 4).

In one example, the vector system could still be stably prepared even when another foreign gene was inserted into the sequence region encoding the SARS-CoV-2 structural proteins. In addition, cells were co-transfected with the vector and a vector expressing an additional antigen, and VLPs containing RNA encoding the additional antigen together with the saRNA could also be produced. Chimeric VLPs in which the receptor-binding domain of the VLP was altered were also stably prepared. Analysis showed that these chimeric VLPs also exhibited markedly enhanced immunogenicity and protective efficacy, indicating that the vector system is useful as a vaccine template against various viruses (see Example 5).

The vaccine composition according to one aspect may be administered through a mucosal route, which is a route of infection for the respiratory disease-causing virus. In addition, because the saRNA in the VLPs contained in the vaccine composition self-amplifies, production of a sufficient amount of antibodies may be induced after a single administration. In addition, since the VLPs do not undergo further propagation, they exhibit a favorable safety profile. Because they induce mucosal immunity, mucosa-specific antibodies are produced, thereby eliciting a markedly enhanced immune response compared to intramuscular injection.

Another aspect provides a method of preventing or treating a respiratory disease, the method including administering a vaccine composition including the virus-like particles to a subject in need thereof.

The terms "vector," "nonstructural protein," "respiratory disease-causing virus," "structural protein," "RNA," "introduction," "virus-like particle," "administration," and the like may have the meanings described above.

In an embodiment, the respiratory disease may be one or more viral infections selected from the group consisting of a coronavirus infection, a human respiratory syncytial virus infection, an adenovirus infection, an influenza virus infection, a rhinovirus infection, an enterovirus infection, a bocavirus infection, a parainfluenza virus infection, a metapneumovirus infection, an Epstein-Barr virus infection, a cytomegalovirus infection, a herpes simplex virus infection, a measles virus infection, and a varicella-zoster virus infection.

In administering the vaccine composition, the vaccine composition may be administered in a volume of 0.1 µL to 0.6 mL per dose, specifically 10 µL to 0.6 mL, and more specifically 30 µL to 0.6 mL.

The vaccine composition may contain the virus-like particles in an amount of 5 µg to 5000 µg, 5 µg to 2500 µg, 5 µg to 1000 µg, 5 µg to 500 µg, 5 µg to 250 µg, 5 µg to 100 µg, 5 µg to 50 µg, 50 µg to 5000 µg, 50 µg to 2500 µg, 50 µg to 1000 µg, 50 µg to 500 µg, 50 µg to 250 µg, 50 µg to 100 µg, 100 µg to 5000 µg, 100 µg to 2500 µg, 100 µg to 1000 µg, 100 µg to 500 µg, 100 µg to 250 µg, 250 µg to 5000 µg, 250 µg to 2500 µg, 250 µg to 1000 µg, 250 µg to 500 µg, 500 µg to 5000 µg, 500 µg to 2500 µg, 500 µg to 1000 µg, 1000 µg to 5000 µg, 1000 µg to 2500 µg, or 2500 µg to 5000 µg per dose of the vaccine composition.

The vaccine composition used in the method of preventing or treating a respiratory disease according to one aspect may be administered through a mucosal route, which is a primary route of viral infection. In addition, because the saRNA in the VLPs contained in the vaccine composition self-amplifies, production of a sufficient amount of antibodies may be induced after a single administration. In addition, since the VLPs do not undergo further propagation, they exhibit a favorable safety profile. Because they induce mucosal immunity, mucosa-specific antibodies are produced, thereby eliciting a markedly enhanced immune response compared to intramuscular injection.

Another aspect provides use of the virus-like particles for the manufacture of a medicament for preventing or treating the respiratory disease.

The terms "vector," "nonstructural protein," "respiratory disease-causing virus," "structural protein," "RNA," "introduction," "virus-like particle," and the like may have the meanings described above.

In an embodiment, the respiratory disease may be one or more viral infections selected from the group consisting of a coronavirus infection, a human respiratory syncytial virus infection, an adenovirus infection, an influenza virus infection, a rhinovirus infection, an enterovirus infection, a bocavirus infection, a parainfluenza virus infection, a metapneumovirus infection, an Epstein-Barr virus infection, a cytomegalovirus infection, a herpes simplex virus infection, a measles virus infection, and a varicella-zoster virus infection.

The medicament for preventing or treating a respiratory disease may be administered at a volume of 0.1 µL to 0.6 mL per single dose, specifically 10 µL to 0.6 mL, and more specifically 30 µL to 0.6 mL.

The medicament for preventing or treating a respiratory disease may include the virus-like particles in an amount of 5 µg to 5000 µg, 5 µg to 2500 µg, 5 µg to 1000 µg, 5 µg to 500 µg, 5 µg to 250 µg, 5 µg to 100 µg, 5 µg to 50 µg, 50 µg to 5000 µg, 50 µg to 2500 µg, 50 µg to 1000 µg, 50 µg to 500 µg, 50 µg to 250 µg, 50 µg to 100 µg, 100 µg to 5000 µg, 100 µg to 2500 µg, 100 µg to 1000 µg, 100 µg to 500 µg, 100 µg to 250 µg, 250 µg to 5000 µg, 250 µg to 2500 µg, 250 µg to 1000 µg, 250 µg to 500 µg, 500 µg to 5000 µg, 500 µg to 2500 µg, 500 µg to 1000 µg, 1000 µg to 5000 µg, 1000 µg to 2500 µg, or 2500 µg to 5000 µg per single dose.

The virus-like particles for the manufacture of a medicament for preventing or treating a respiratory disease according to one aspect may be administered through a mucosal route, the primary infection route of the virus. In addition, the saRNA within the VLPs can self-amplify and drive production of an amount of antigen sufficient to elicit immunity after a single administration. In addition, since the VLPs do not undergo further propagation, they exhibit a favorable safety profile. Because they induce mucosal immunity, mucosa-specific antibodies are produced, thereby eliciting a markedly enhanced immune response compared to intramuscular injection of the VLPs.

### Advantageous Effects of Invention

The present disclosure relates to a vector for producing virus-like particles (VLPs), VLPs produced using the vector, and a vaccine composition containing the VLPs, wherein the vector is a single-vector system that enables easy and convenient production of saRNA-containing VLPs without a helper vector. The VLPs produced using the vector and the vaccine composition containing the VLPs can be administered through a mucosal route, which is a primary route of viral infection, and because the saRNA in the VLPs self-amplifies, production of a sufficient amount of antibodies can be induced after a single administration. In addition, because the VLPs do not undergo further propagation, they exhibit excellent safety and, as a form suitable for mucosal administration, can induce mucosal immunity and thereby exhibit markedly superior prophylactic or therapeutic effects against a respiratory disease compared with an intramuscularly administered therapeutic agent.

### Brief Description of Drawings

FIG. 1 shows expression of conventional mRNA and self-amplifying RNA (saRNA) after *in vivo* administration.
FIG. 2 shows structures of a vector for preparing VLPs containing saRNA, RNA expressed from the vector, and prepared VLPs.
FIG. 3 shows configuration of a vector for preparing VLPs containing saRNA.
FIG. 4 shows Western blot results for RNAs transcribed from respective vectors into which sequences encoding SARS-CoV-2 spike, envelope, membrane, and nucleocapsid proteins had been inserted, and Western blot results for RNAs expressed from a vector into which sequences encoding SARS-CoV-2 spike, envelope, and membrane proteins had been inserted and a vector into which a sequence encoding nucleocapsid had been inserted.
FIG. 5 shows immunofluorescence assay results confirming proteins produced in cells transfected with RNA prepared by linearizing a vector.
FIG. 6 shows Western blot results for transfected-cell lysates and supernatants, confirming VLP production.
FIG. 7 shows evaluation of VLP production in a HEK 293-N cell line.
FIG. 8 shows Western blot results for RNA transcribed from a vector into which sequences encoding SARS-CoV-2 spike, envelope, and membrane proteins had been inserted.
FIG. 9 shows immunofluorescence assay results confirming proteins produced after saRNA transfection into a HEK 293-N cell line.
FIG. 10 shows Western blot results for transfected-cell lysates and supernatants, confirming VLP production.
FIG. 11 is a microscopy image of a large-scale VLP-production cell line expressing nucleocapsid protein.
FIG. 12 is a TEM image showing VLP morphology.
FIG. 13 shows VLP particle-size distribution.
FIG. 14 shows evaluation of VLP infectivity in BHK-21, HACE2-293T, and A549-HACE2-TMPRSS2 cell lines.
FIG. 15 shows microscopy images confirming VLP infectivity in HACE2-293T and A549-HACE2-TMPRSS2 cell lines.
FIG. 16 shows immunofluorescence assay results confirming SARS-CoV-2 spike protein expression in human bronchial epithelial cells.
FIG. 17 shows ISH results confirming SARS-CoV-2 spike gene expression in human bronchial epithelial cells.
FIG. 18 shows Western blot results for lysates of cells infected with VLPs.
FIG. 19 shows TEM images of a VERO E6 cell line at 30 minutes and 48 hours after VLP infection.
FIG. 20 shows SARS-CoV-2 spike gene expression in mouse lungs after intranasal VLP inoculation.
FIG. 21 shows SARS-CoV-2 membrane protein expression in mouse lungs after intranasal VLP inoculation.
FIG. 22 is a schematic diagram illustrating a VLP vaccine administration experiment in BALB/c mice.
FIG. 23 shows titers of α-spike RBD IgG and α-spike trimer IgG in mice after VLP vaccination.
FIG. 24 shows titers of α-spike RBD IgA and α-spike trimer IgA in mice after VLP vaccination.
FIG. 25 shows neutralizing antibody titers in mice after VLP vaccination.
FIG. 26 shows INF-γ secretion levels in mice after VLP vaccination.
FIG. 27 shows expression levels of INF-α and INF-β genes in mice after VLP vaccination.
FIG. 28 shows numbers of INF-γ⁺ CD4⁺ T cells in mice after VLP vaccination.
FIG. 29 shows changes in mouse body weight following viral challenge after VLP vaccination.
FIG. 30 shows mouse survival rates following viral challenge after VLP vaccination.
FIG. 31 shows changes in viral titer in mouse lungs following viral challenge after VLP vaccination.
FIG. 32 shows changes in mouse lung function following viral challenge after VLP vaccination.
FIG. 33 is a schematic diagram illustrating a VLP vaccine administration experiment in hACE2-Tg mice.
FIG. 34 shows titers of α-spike RBD IgG and α-spike trimer IgG in mice after VLP vaccination.
FIG. 35 shows titers of α-spike RBD IgA and α-spike trimer IgA in mice after VLP vaccination.
FIG. 36 shows neutralizing antibody titers in mice after VLP vaccination.
FIG. 37 shows INF-γ secretion levels in mice after VLP vaccination.
FIG. 38 shows expression levels of INF-α and INF-β genes in mice after VLP vaccination.
FIG. 39 shows changes in mouse body weight after VLP vaccination.
FIG. 40 shows changes in mouse body weight following viral challenge after VLP vaccination.
FIG. 41 shows mouse survival rates following viral challenge after VLP vaccination.
FIG. 42 shows qPCR measurement results for virus present in mouse lungs and nasal cavities following viral challenge after VLP vaccination.
FIG. 43 shows changes in mouse lung function following viral challenge after VLP vaccination.
FIG. 44 shows foreign protein expression in cells transfected with a vector into which foreign gene LacZ had been inserted.
FIG. 45 is a schematic diagram showing configuration of each vector and structure of prepared VLPs when a vector expressing an additional antigen (luciferase) is co-introduced into cells together with a vector system for VLP preparation.
FIG. 46 shows expression levels of NSP1, spike, and luciferase genes when a vector expressing an additional antigen (luciferase) is co-introduced into cells together with a vector system for VLP preparation.
FIG. 47 shows images confirming additional-antigen expression in SARS2 JN.1-infected mice and uninfected mice when a vector expressing an additional antigen (luciferase) is co-introduced into cells together with a vector system for VLP preparation.
FIG. 48 is a graph confirming additional-antigen expression in SARS2 JN.1-infected mice and uninfected mice when a vector expressing an additional antigen (luciferase) is co-introduced into cells together with a vector system for VLP preparation.
FIG. 49 shows VLPs generated by replacing RBD of SARS-CoV-2 Omicron BA.1 spike protein with RBD of SARS-CoV-2 Omicron XBB.
FIG. 50 shows VLPs generated by replacing RBD of SARS-CoV-2 Omicron BA.1 spike protein with RBD of MERS-CoV.
FIG. 51 shows preparation of MERS-CoV chimeric VLPs in a large-scale production cell line.
FIG. 52 shows expression levels of genes encoding envelope and membrane proteins of MERS-CoV chimeric VLPs in a large-scale VLP-production cell line.
FIG. 53 is a schematic diagram illustrating an Omicron XBB.1 chimeric VLP vaccine administration experiment in BALB/c mice.
FIG. 54 shows neutralizing antibody titers in mice after Omicron XBB.1 chimeric VLP vaccination.
FIG. 55 shows expression levels of INF-α and INF-β genes in mice after Omicron XBB.1 chimeric VLP vaccination.
FIG. 56 shows changes in viral titer in mouse lungs following viral challenge after Omicron XBB.1 chimeric VLP vaccination.
FIG. 57 is a schematic diagram illustrating an Omicron XBB.1 chimeric VLP vaccine administration experiment in hACE2-Tg mice.
FIG. 58 shows neutralizing antibody titers in mice after Omicron XBB.1 chimeric VLP vaccination.
FIG. 59 shows expression levels of INF-α and INF-β genes in mice after Omicron XBB.1 chimeric VLP vaccination.
FIG. 60 shows changes in mouse body weight following viral challenge after Omicron XBB.1 chimeric VLP vaccination.
FIG. 61 shows changes in mouse survival rates following viral challenge after Omicron XBB.1 chimeric VLP vaccination.
FIG. 62 shows changes in viral titer in mouse lungs following viral challenge after Omicron XBB.1 chimeric VLP vaccination.
FIG. 63 shows expression of MERS-CoV RBD protein when a VERO E6 cell line was infected with MERS-CoV chimeric VLPs.
FIG. 64 is a schematic diagram illustrating a MERS-CoV chimeric VLP vaccine administration experiment in hDPP4 mice.
FIG. 65 shows IgG titers in mice after MERS-CoV chimeric VLP vaccination.
FIG. 66 shows neutralizing antibody titers in mice after MERS-CoV chimeric VLP vaccination.
FIG. 67 shows changes in mouse body weight following viral challenge after MERS-CoV chimeric VLP vaccination.
FIG. 68 shows mouse survival rates following viral challenge after MERS-CoV chimeric VLP vaccination.
FIG. 69 shows changes in viral titer in mouse lungs following viral challenge after MERS-CoV chimeric VLP vaccination.

### Best Mode for Carrying out the Invention

Hereinafter, the present disclosure will be described in more detail with reference to the following Examples. However, these Examples are provided for illustrative purposes only, and the scope of the present disclosure is not limited thereto.

### Reference Examples

### <Reference Example 1> SARS-CoV-2 VLP Infection

VERO E6, HEK-293T, A549-HACE2-TMPRSS2, and BHK-21 cells were infected with 20-fold diluted VLPs (SARS-CoV-2 or MERS). After 1 hour, the medium was replaced with DMEM containing 2 % FBS, and the cells were maintained for 48 hours. At 48 hours post-infection (hpi), the cells were washed three times with warm PBS, and viral and host RNAs were extracted from the cell lysates and supernatants using a Maxwell RSC Viral Total Nucleic Acid Purification Kit (Promega, WI).

### <Reference Example 2> Quantitative RT-PCR

The RNA yield was evaluated through an RT-qPCR analysis. Specifically, cDNA was amplified in a StepOnePlus Real-Time PCR system (QuantStudio 5; Thermo Fisher Scientific) using a reaction mixture containing a 1× One Step TB Green PrimeScript RT-PCR kit (Takara) in the presence of primers specifically targeting an internal viral sequence common to all viral transcripts (N amplicon) or subgenomic N mRNA. The reaction was performed at 42 °C for 5 minutes and 95 °C for 10 seconds, followed by 40 cycles of 95 °C for 5 seconds and 60 °C for 30 seconds.

### <Reference Example 3> ELISA and Neutralizing Antibody Assay

A 96-well NUNC-MaxiSorp plate was coated overnight at 4 °C with SARS-CoV-2 Omicron BA.1 RBD (Sinobiological, #40592-V08H121) or full-length spike trimer (Sinobiological, #40589-V08H26) diluted to 1 µg/mL in PBS (pH 7.4). The plate was blocked with a blocking buffer (PBS containing 5 % BSA and 0.05 % Tween-20) at 37 °C for 1 hour.

The samples were serially diluted, starting with a 1:10 dilution, using a reagent diluent (PBS containing 1 % BSA and 0.05 % Tween-20). After incubation at 37 °C for 2 hours, the plate was washed four times with a wash buffer (PBS containing 0.05 % Tween-20), and then a 1:20,000 dilution of goat anti-mouse IgG-HRP (Abcam, #ab97023) was added, followed by incubation at 37 °C for 1 hour.

For the serum neutralization assay, Vero E6 cells suspended in DMEM (supplemented with 10 % FBS, 1 % L-glutamine, and 100 U/mL penicillin-streptomycin) were seeded into a 96-well plate at a density of 3 × 10⁴ cells per well. After 24 hours, the serum was inactivated by incubation at 56 °C for 30 minutes and then diluted 1:10 in DMEM (without FBS, containing 1 % L-glutamine and 100 U/mL penicillin-streptomycin), followed by 1:2 serial dilutions in the 96-well plate. Then, an equal volume of SARS-CoV-2 containing 100 TCID₅₀ per well was added to the diluted serum. The serum-virus mixture was incubated at 37 °C for 1 hour and then added to the Vero E6 cells. Subsequently, the Vero E6 culture medium was replaced with 150 µL of low-serum DMEM (containing 2 % FBS, 1 % L-glutamine, and 100 U/mL penicillin-streptomycin), and the cells were incubated at 37 °C for 72 hours to observe cytopathic effects.

### <Reference Example 4> Animal Experiments

Animal experiments were performed in accordance with the approval of the Institutional Animal Care and Use Committee of the Institute for Basic Science (IBS-2023-039). To investigate the efficacy of the SARS-CoV-2 VLPs (XBB.1 chimeric VLP or BA.1 VLP) as vaccines, 8- to 10-week-old female BALB/c or hACE2-Tg mice were randomly divided into groups of 13 mice each. To investigate the efficacy of the MERS VLP as a vaccine, 8- to 10-week-old female hDPP4 mice were randomly divided into groups of 10 mice each. Following anesthesia, the mice were intranasally inoculated with 30 µL of the VLPs or PBS.

For the SARS-CoV-2 VLPs, at 4 weeks post-inoculation, the mice were intranasally inoculated with 30 µL of 1 LD₅₀ of Wuhan MA or XBB.1. Body weight and survival rate were monitored for approximately 14 days, and at 3, 5, and 7 days post-infection (dpi), two mice per group were euthanized to harvest nasal turbinates and lung tissues for histopathological examination and qRT-PCR.

For the MERS VLP, a booster dose of the MERS VLP was administered at 5 weeks post-inoculation. At 2 weeks post-boost, the mice were intranasally inoculated with 30 µL of 10 LD₅₀ of MERS-CoV. Body weight and survival rate were monitored for approximately 14 days, and at 3, 5, and 7 dpi, two mice per group were euthanized to harvest nasal turbinates and lung tissues for histopathological examination and qRT-PCR.

### <Reference Example 5> Lung Histopathology

Immunohistochemistry (IHC) was performed using a Bond RXm (Leica) automated platform. Specifically, the mouse lung slides were deparaffinized, rehydrated, and then pretreated with an epitope retrieval ER2 high-pH buffer (Leica). Endogenous peroxidase was inactivated with H₂O₂, and nonspecific signals were blocked with mouse serum (1/500 dilution). A rabbit anti-SARS-CoV-2 nucleocapsid primary antibody (Abcam, #ab271180) was applied at a dilution of 1:200, followed by treatment with a peroxidase-conjugated anti-rabbit IgG (Jackson ImmunoResearch, #111-035-003) at a 1:1000 dilution.

Immunoreactivity was visualized via a diaminobenzidine chromogen reaction. The slides were counterstained with hematoxylin, sequentially dehydrated using ethanol, cleared with xylene, and permanently mounted. A negative control was prepared by substituting the primary antibody with a BOND Primary Antibody Diluent (Leica, #AR9352). Microscopy images at a 40× magnification were acquired using an Axio Scan Z1.

In situ hybridization (ISH) was performed using an RNAscope 2.5 HD Detection Reagent-RED Kit (ACDBio, MN) in accordance with the manufacturer's instructions. Specifically, the lung slides were deparaffinized, treated with H₂O₂, and then pretreated with an antigen retrieval solution. An nCoV2019-S probe (ACDBio, #848561) was hybridized for 2 hours. Signals were amplified using Amp 1-6 solutions and detected using a RED solution. The slides were counterstained with hematoxylin and mounted, and slide images at a 40× magnification were acquired using the Axio Scan Z1.

### Preparation Examples

### <Preparation Example 1> Preparation of Vectors

The pSP6-26s plasmid was constructed by removing sequences encoding the nonstructural and structural proteins of Semliki Forest virus (SFV) from the pSFV3 backbone (Addgene #92072). Sequences encoding the structural proteins of SARS-CoV-2 Omicron BA.1, together with a packaging signal sequence, were synthesized (Bionics, Korea) and subcloned into the pSP6-26s and pcDNA3.1(+) (GenScript, NJ) plasmids. The replicon plasmid pSFV3-SEM-PS9 was constructed by subcloning sequences encoding the SARS-CoV-2 Omicron BA.1 structural proteins (spike protein, envelope protein, and membrane protein), together with a packaging signal sequence, into a pSFV3 backbone (containing sequences encoding Semliki Forest virus nonstructural proteins NSP1 to NSP4).

The vectors for preparing chimeric VLPs were constructed by replacing only the receptor binding domain (RBD) portion of the SARS-CoV-2 Omicron BA.1 spike protein in the pSFV3-SEM-PS9 with the RBD of SARS-CoV-2 Omicron XBB.1 or MERS-CoV, respectively.

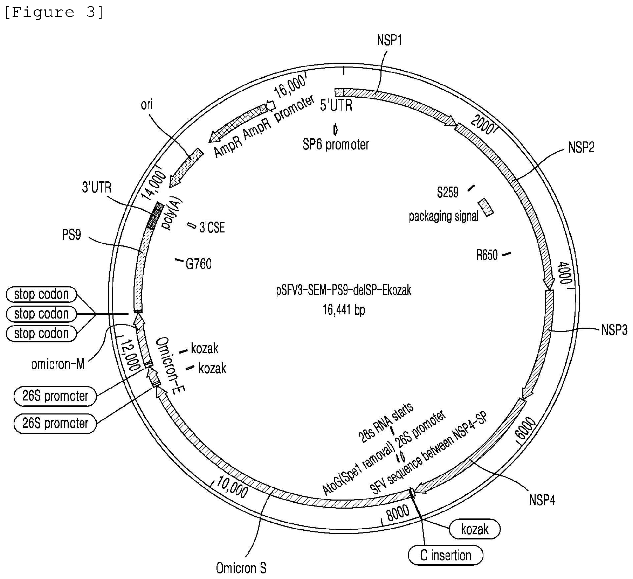

For the packaging signal sequence of SARS-CoV-2, the sequence of SEQ ID NO: 20 or a gene sequence encoding a portion of NSP11 to NSP12 of the SARS-CoV-2 BA.1 strain was used. Details of the inserted sequences are as shown in Table 1 below, and the configurations of the vectors are as illustrated in FIG. 3.

**[Table 1]**

| SEQ ID NO. | Sequence Name |
|---|---|
| SEQ ID NO: 1 | Semliki Forest virus (SFV) NSP1 (DNA) |
| SEQ ID NO: 2 | SFV NSP1(RNA) |
| SEQ ID NO: 3 | SFV NSP2(DNA) |
| SEQ ID NO: 4 | SFV NSP2(RNA) |
| SEQ ID NO: 5 | SFV NSP3(DNA) |
| SEQ ID NO: 6 | SFV NSP3(RNA) |
| SEQ ID NO: 7 | SFV NSP4(DNA) |
| SEQ ID NO: 8 | SFV NSP4(RNA) |
| SEQ ID NO: 9 | SFV RdRp(DNA) |
| SEQ ID NO: 10 | SFV RdRp(RNA) |
| SEQ ID NO: 11 | SARS-CoV-2(BA.1) spike gene (DNA) |
| SEQ ID NO: 12 | SARS-CoV-2(BA.1) spike gene (RNA) |
| SEQ ID NO: 13 | SARS-CoV-2(BA.1) envelope gene (DNA) |
| SEQ ID NO: 14 | SARS-CoV-2(BA.1) envelope gene (RNA) |
| SEQ ID NO: 15 | SARS-CoV-2(BA.1) membrane gene (DNA) |
| SEQ ID NO: 16 | SARS-CoV-2(BA.1) membrane gene (RNA) |
| SEQ ID NO: 17 | SARS-CoV-2(BA.1) nucleocapsid gene (DNA) |
| SEQ ID NO: 18 | SARS-CoV-2(BA.1) nucleocapsid gene (RNA) |
| SEQ ID NO: 19 | SARS-CoV-2(BA.1) packaging signal 1 (DNA) |
| SEQ ID NO: 20 | SARS-CoV-2(BA.1) packaging signal 1 (RNA) |
| SEQ ID NO: 21 | saRNA expressed from pSFV3-SEM-PS9 |
| SEQ ID NO: 22 | pSFV3-SEM-PS9 |
| SEQ ID NO: 23 | SARS-CoV-2(XBB.1 RBD) chimeric spike gene (DNA) |
| SEQ ID NO: 24 | SARS-CoV-2(XBB.1 RBD) chimeric spike gene (RNA) |
| SEQ ID NO: 25 | MERS-CoV(MERS RBD) chimeric spike gene (DNA) |
| SEQ ID NO: 26 | MERS-CoV(MERS RBD) chimeric spike gene (RNA) |

### <Preparation Example 2> Cell Culture for VLP Production

HEK 293 cells (*Homo sapiens* kidney epithelial cells; ATCC CRL-1573) were cultured in DMEM (Thermo Fisher Scientific) supplemented with 10 % heatinactivated fetal bovine serum (FBS; Thermo Fisher Scientific), penicillin (100 U/mL), and streptomycin (100 µg/mL), which are antibiotics. The cells were maintained and cultured at 37 °C in a 5 % CO₂ atmosphere.

HEK 293 cells expressing the nucleocapsid (N) protein (HEK 293-N cells) were obtained by transfecting HEK 293 cells with a linearized pcDNA3.1(+)-N plasmid using Lipofectamine 3000 (Thermo Fisher Scientific). After 2 days, the cells were subjected to clonal selection with 1 mg/mL G418 (InvivoGen), and nucleocapsid expression was examined by an immunofluorescence assay.

### <Preparation Example 3> Preparation of SARS-CoV-2 VLPs Containing saRNA

RNA transcripts were obtained through an in vitro transcription method using a MEGAscript SP6 Kit (Invitrogen). Next, 400 µg of the replicon RNA was added to a 4 mm cuvette containing a solution of HEK 293-N cells (containing 8 × 10⁶ cells) suspended in 0.8 mL of DMEM. A single electrical pulse was applied using a GenePulser X (Bio-Rad) set to 260 V and 550 µF. The cells were transferred to a 100 mm culture dish, after which VLPs were produced. At 48 hours post-transfection, the culture medium containing the VLPs was harvested and centrifuged at 3,000 rpm for 10 minutes. The supernatant was filtered through a 0.45 µm syringe filter.

To concentrate and purify the SARS-CoV-2 VLPs, the filtrate was layered onto a 20 % sucrose cushion and subjected to ultracentrifugation at 30,000 rpm for 4 hours at 4 °C using an SW32Ti rotor (Beckman Coulter). Thereafter, the obtained pellet was suspended in a PBS solution, and residual sucrose and proteins were removed by centrifugation at 4,000 rpm for 1 hour at 4 °C using an Amicon Ultra-2mL-30K (Merck). The purified VLPs were finally suspended in PBS and stored at -80 °C until use.

### Examples

### <Example 1> Confirmation of Production of VLPs Containing saRNA

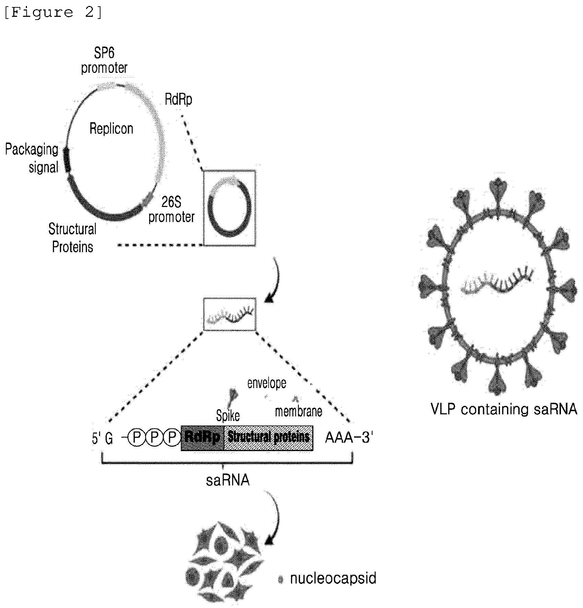

To determine whether a vector for preparing VLPs, into which a SARS-CoV-2 packaging sequence and sequences encoding SARS-CoV-2 structural proteins had been inserted, could stably produce VLPs when introduced into cells, i.e., whether self-amplifying RNA (saRNA) was transcribed based on the sequences inserted in the vector and VLPs were generated, the replicon plasmid vector (pSFV3-SEM-PS9, FIG. 3) was subjected to *in vitro* transcription (IVT) in accordance with Preparation Examples 1 to 3 to prepare saRNA. The saRNA was then transfected into a cell line to prepare VLPs (FIG. 2), and the prepared VLPs were analyzed by Western blotting and an immunofluorescence assay.

### (1) Evaluation of Stability of the Vector System in BHK-21 Cell Line

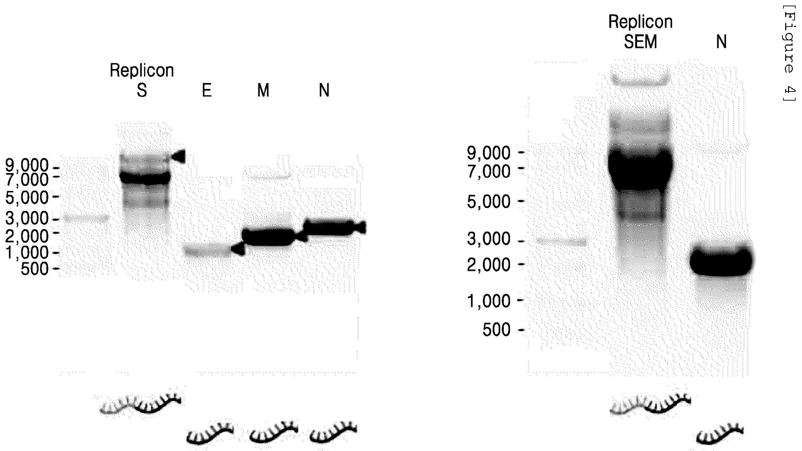

As shown in FIG. 4, IVT was performed on replicon plasmid vectors in which a SARS-CoV-2 Omicron BA.1 packaging signal sequence and either sequences encoding the respective structural proteins spike (S), envelope (E), membrane (M), and nucleocapsid (N), or sequences encoding the spike, envelope, and membrane proteins, were inserted into the pSFV3 backbone in place of the sequence encoding the structural proteins of Semliki Forest virus (SFV). As a result, respective bands were detected that corresponded to the mRNAs for the SARS-CoV-2 structural proteins (S, E, M, and N) and to the mRNA containing sequences encoding the spike, envelope, and membrane proteins. These results indicated that mRNA transcripts were generated from the replicon plasmid vectors by IVT.

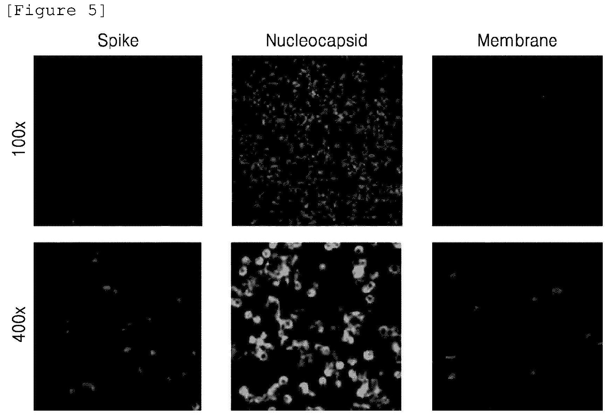

As shown in FIG. 5, after the mRNA prepared by IVT was transfected into the BHK-21 cell line, proteins produced in the cells were examined by an immunofluorescence assay. The assay showed that the spike, membrane, and nucleocapsid proteins, which are structural proteins of SARS-CoV-2, were expressed in the cells.

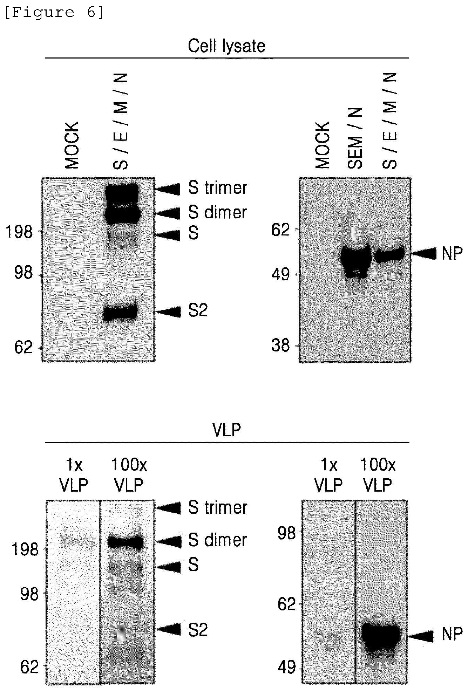

In addition, cell lysates were prepared from the transfected cells, and VLPs released into the culture supernatant were purified. These samples were then subjected to Western blotting. The results showed that VLPs were formed both when mRNAs encoding the respective SARS-CoV-2 structural proteins (S, E, M, and N) were transfected and when mRNA containing sequences encoding the spike, envelope, and membrane proteins was transfected. In particular, VLPs were produced more efficiently when the mRNA containing sequences encoding the spike, envelope, and membrane proteins was transfected (FIG. 6).

These results demonstrated that VLPs were stably generated when cells were transfected with RNA generated from the vector system including a SARS-CoV-2 Omicron BA.1 packaging signal sequence together with sequences encoding the spike, envelope, and membrane proteins, which are structural proteins of SARS-CoV-2 Omicron BA.1.

### (2) Evaluation of Stability of the Vector System in HEK 293-N Cell Line

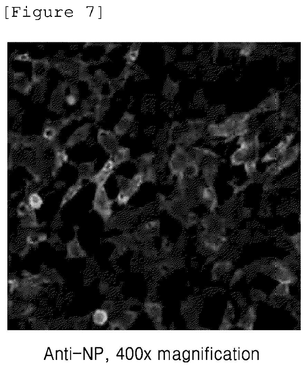

As shown in FIG. 7, a cell line stably and constitutively expressing the nucleocapsid protein (HEK 293-N cell line) was established. It was further examined whether VLPs were generated when cells were transfected with RNA generated from a replicon plasmid vector (pSFV3-SEM-PS9) in which a SARS-CoV-2 Omicron BA.1 packaging signal sequence and sequences encoding the SARS-CoV-2 Omicron BA.1 structural proteins (S,E, and M) other than the nucleocapsid protein had been inserted into the pSFV3 backbone in place of sequences encoding the structural proteins of SFV.

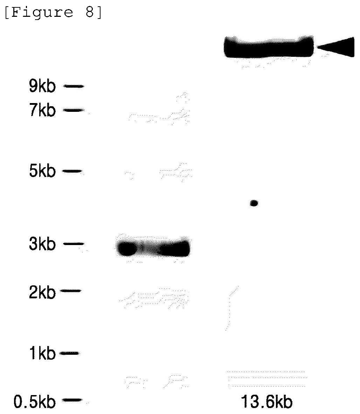

As shown in FIG. 8, when the replicon plasmid vector (pSFV3-SEM-PS9) was subjected to IVT, a band (at approximately 13,000 bp) corresponding to the target saRNA was detected.

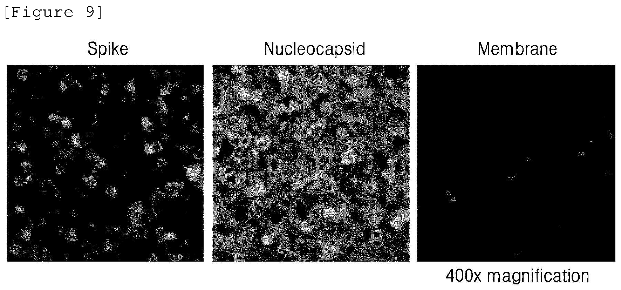

As shown in FIG. 9, after the saRNA prepared by IVT was transfected into the HEK 293-N cell line, proteins produced in the cells were examined by an immunofluorescence assay. The assay showed that the SARS-CoV-2 structural proteins (the spike, nucleocapsid, and membrane proteins) were expressed in the cells.

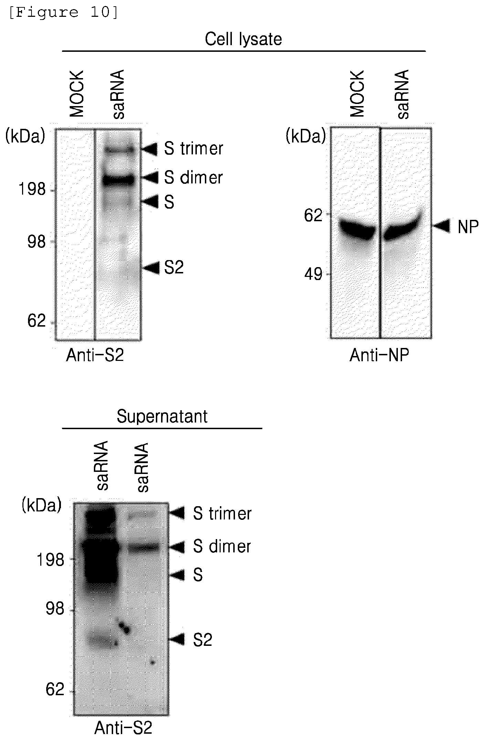

In addition, cell lysates were prepared from the transfected cells, and VLPs released into the culture supernatant were purified. These samples were then subjected to Western blotting. The results showed that SARS-CoV-2 VLPs were formed when saRNA containing sequences encoding the SARS-CoV-2 spike, envelope, and membrane structural proteins was transfected (FIG. 10).

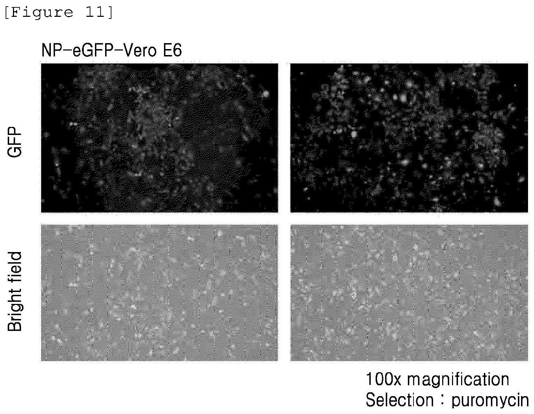

These results demonstrated that HEK 293-N cell line constitutively expressing the nucleocapsid protein also stably produced VLPs through the vector system upon transfection with saRNA generated from the replicon plasmid vector system (pSFV3-SEM-PS9). Accordingly, using the Vero E6 cell line, a large-scale VLP-producing cell line expressing the SARS-CoV-2 nucleocapsid protein was prepared to enable repeated production of VLPs (FIG. 11).

### <Example 2> Characterization of VLPs Containing saRNA

To characterize VLPs containing saRNA, the structure and size of the VLPs prepared according to Preparation Examples 1 to 3 were evaluated.

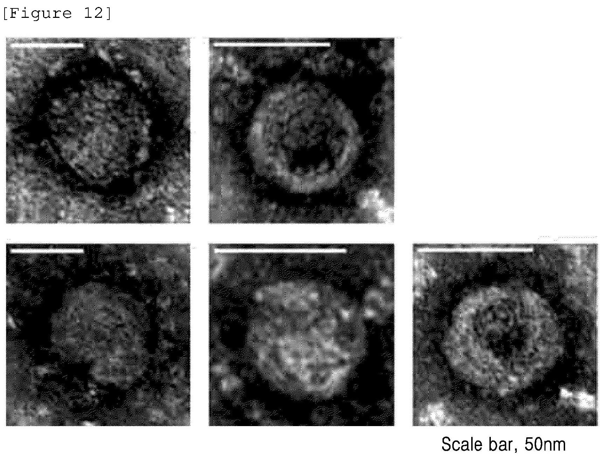

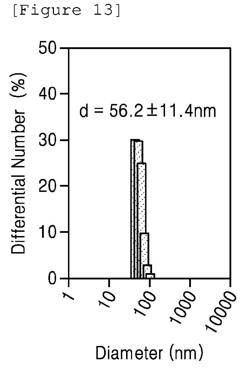

As shown in FIG. 12, the structure of VLPs containing saRNA containing sequences encoding SARS-CoV-2 structural proteins and RdRp and including a packaging sequence was examined by transmission electron microscopy (TEM). The VLPs were observed to have irregular morphology. As shown in FIG. 13, the size of the VLPs was measured by dynamic light scattering (DLS), and the VLPs had a diameter of approximately 56 nm.

Measurement of the amount of the VLPs produced in the VLP-producing cell line (HEK 293-N) showed that, as shown in Table 2 below, 10.6 Log₁₀ copies/mL or more of VLPs were produced at all sites, confirming that a sufficient amount of VLPs for inoculation was stably produced through the vector system.

**[Table 2]**

| Log₁₀ copies/mL | Site 1 | Site 2 | Site 3 | Site 4 | Site 5 | Site 6 |
|---|---|---|---|---|---|---|
| | 12.3 | 10.6 | 12.5 | 12.2 | 12.3 | 11.9 |

### <Example 3> Evaluation of In Vitro Cell Infectivity of VLPs Containing saRNA

To determine the cell infectivity of VLPs containing saRNA, the infectivity of the VLPs prepared according to Preparation Examples 1 to 3 was evaluated in cells having hACE2 and TMPRSS2 receptors (2D culture) and in human bronchial epithelial cells (3D culture) by Western blotting, an immunofluorescence assay, and ISH. In addition, TEM images and Western blot analysis of VLP-infected cells were used to examine whether reinfection occurred after VLP infection.

### (1) Evaluation of In Vitro Cell Infectivity of VLPs Containing saRNA

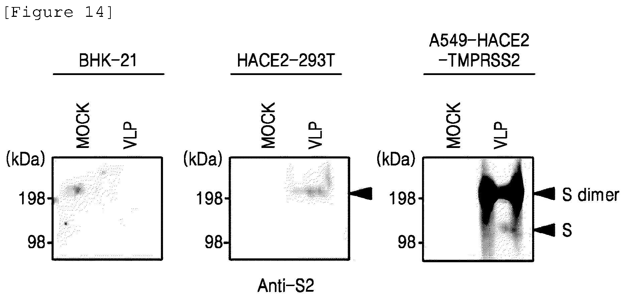

As shown in FIG. 14, VLPs containing saRNA showed infectivity in the cell line (HACE2-293T), which expresses hACE2, a host-cell receptor used by SARS-CoV-2 for host-cell entry, and in the cell line (A549-HACE2-TMPRSS2), which expresses hACE2 together with the host protease TMPRSS2.

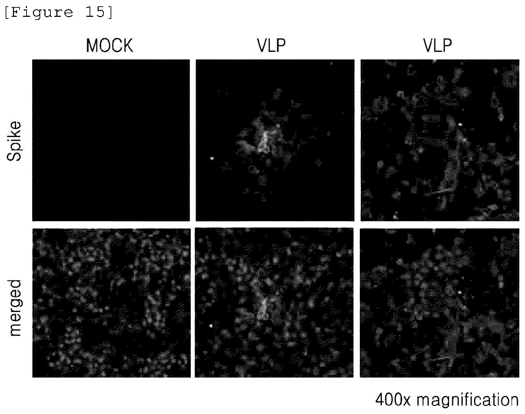

In addition, as shown in FIG. 15, when the cell line expressing hACE2 and TMPRSS2 was infected with the VLPs, expression of the SARS-CoV-2 spike protein was detected, confirming that the VLPs containing saRNA were capable of infecting cells.

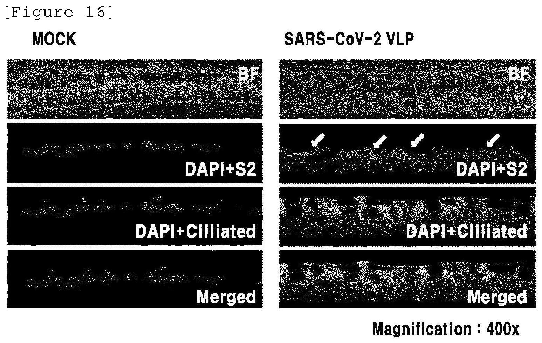

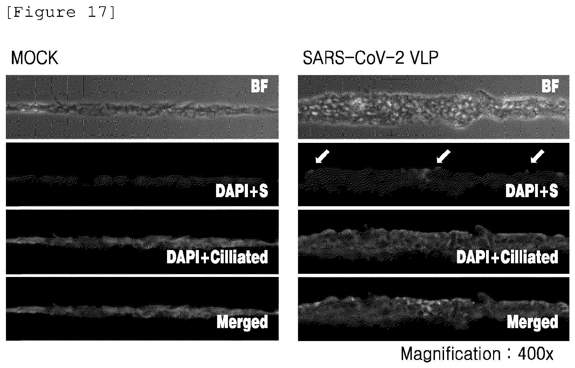

As shown in FIGS. 16 and 17, VLP infectivity in 3D-cultured human bronchial epithelial cells was examined. An immunofluorescence assay showed that the SARS-CoV-2 spike protein was expressed in the human bronchial epithelial cells (FIG. 16). In addition, ISH showed that the SARS-CoV-2 spike gene was expressed in the human bronchial epithelial cells, and the ISH results also showed that saRNA was distributed within the VLPs (FIG. 17).

### (2) Evaluation of Reassembly Potential of VLPs Containing saRNA

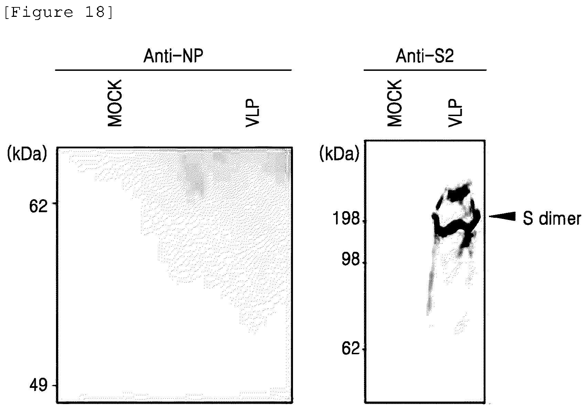

To determine whether reassembly of VLPs containing saRNA occurred after infection, Western blotting was performed on lysates of cells infected with the VLPs. As shown in FIG. 18, the culture supernatant collected 2 days after infection of A549-HACE2-TMPRSS2 cells with VLPs containing saRNA was examined. No nucleocapsid protein of the VLPs containing saRNA was detected in the culture supernatant, indicating that the VLPs were not reassembled after cell infection.

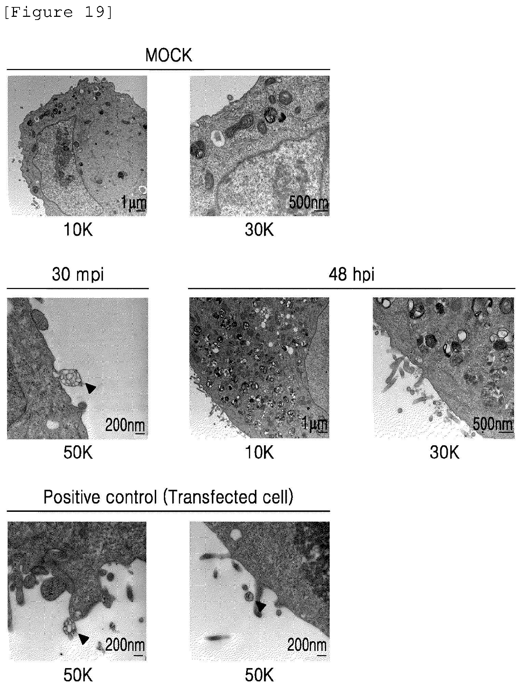

In addition, as shown in FIG. 19, the VERO E6 cell line was infected with VLPs, and the cells were examined by TEM after 30 minutes and 48 hours. At 30 minutes, the cells were observed to be infected with the VLPs, whereas no VLPs were detected in the cells after 48 hours. These results indicated that the VLPs were not reassembled after a single infection.

That is, after VLPs containing saRNA infect host cells, the saRNA carried by the VLPs directs expression of SARS-CoV-2 structural proteins, thereby forming SARS-CoV-2 VLPs that do not contain saRNA. Accordingly, the VLPs exhibit high immunogenicity after a single infection and, because reassembly of saRNA-containing VLPs was not observed, the likelihood of additional infection is markedly low, indicating excellent safety.

### <Example 4> Evaluation of In Vivo Infectivity of VLPs Containing saRNA in Mice

To determine the infectivity of VLPs containing saRNA in mice, VLPs prepared according to Preparation Examples 1 to 3 were administered to mice prepared according to Reference Example 4, namely hACE2-Tg mice and BALB/c mice. VLP infectivity in mouse lung tissue, as well as immunogenicity and protective efficacy in mice following VLP administration, was then evaluated.

### (1) Evaluation of Infectivity of VLPs Containing saRNA

In accordance with Reference Example 5, immunohistochemistry (IHC) and in situ hybridization (ISH) analyses were performed on lung tissue samples from hACE2-Tg mice that received intranasal administration of VLPs containing saRNA, to determine whether VLP infection occurred.

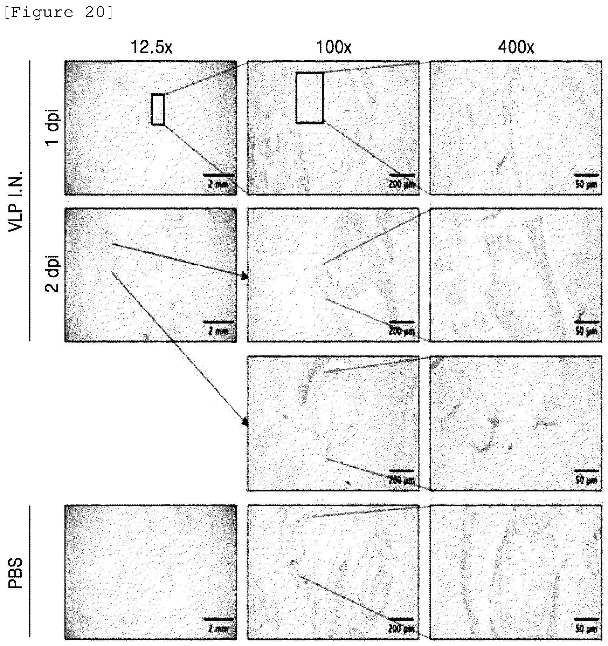

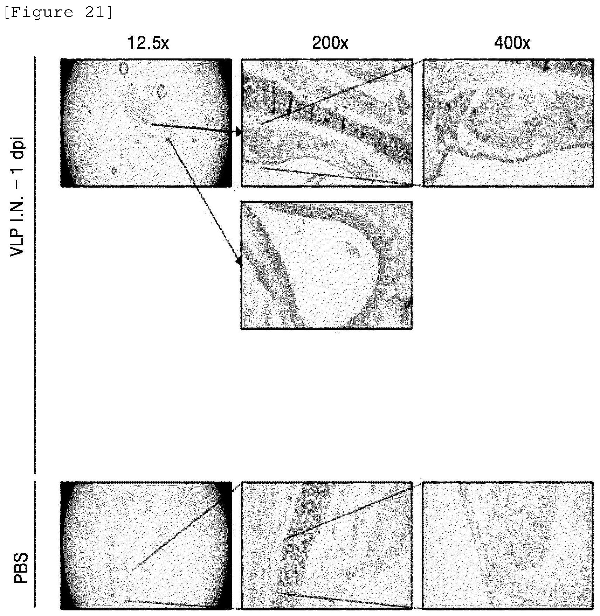

As shown in FIG. 20, after the VLP vaccine containing saRNA was administered intranasally, expression of the SARS-CoV-2 spike gene was detected in the lungs of the mice on days 1 and 2 post-inoculation. In addition, as shown in FIG. 21, expression of the SARS-CoV-2 membrane protein was detected in mouse lung tissue on day 1 after vaccine inoculation.

Based on these results, the VLP vaccine containing saRNA was shown to exhibit high infectivity in mice.

### (2) Evaluation of Immunogenicity and Protective Efficacy (BALB/c Mice)

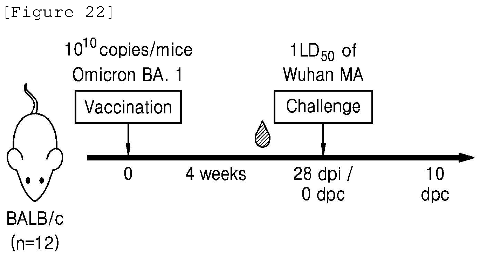

After the VLP vaccine containing saRNA containing a sequence encoding the structural proteins of Omicron BA.1 was administered once intranasally (I.N.) at 10¹⁰ copies/mouse, blood was collected from the mice. The mice were then challenged with 1 LD₅₀ of Wuhan MA virus, and blood samples were collected over 10 days to monitor the course of infection (FIG. 22).

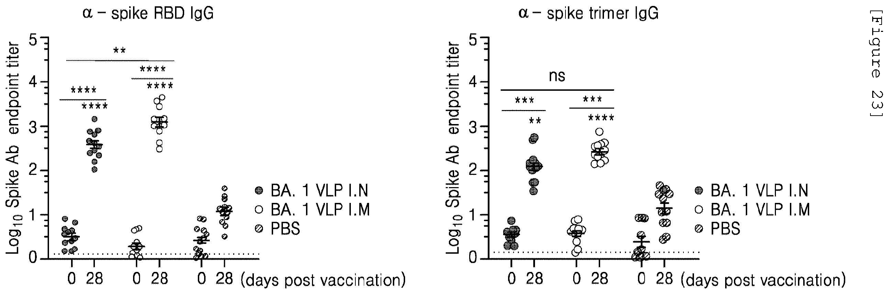

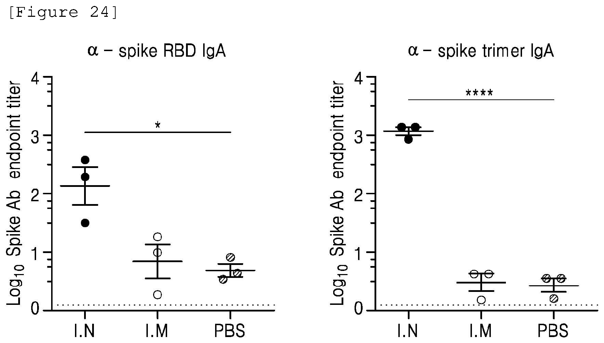

At 28 days after inoculation with the VLP vaccine containing saRNA, both α-spike RBD IgG and α-spike trimer IgG levels were increased compared with the control group (PBS) (FIG. 23). For α-spike RBD IgA and α-spike trimer IgA, which indicate mucosal immunity, significantly greater increases were observed in the group receiving intranasal administration of the VLP vaccine containing saRNA than in the control group and the group receiving intramuscular (I.M.) administration of the vaccine. That is, for IgA, which is a mucosal immune antibody, the antibody titer was significantly higher only in the intranasal administration group. These results indicated that intranasal administration of the vaccine induced markedly enhanced antibody production (FIG. 24).

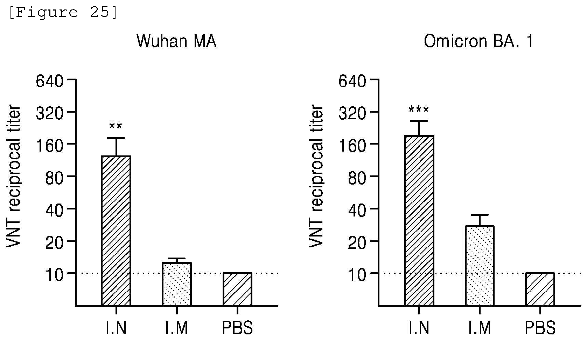

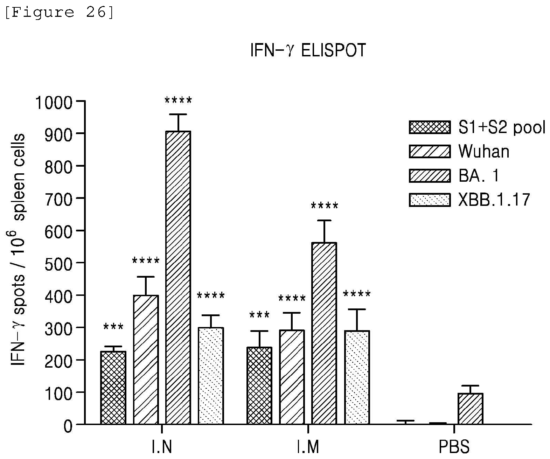

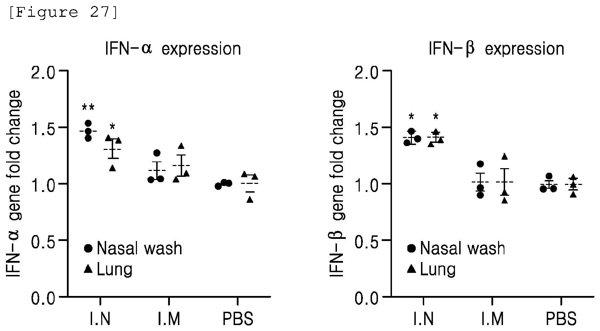

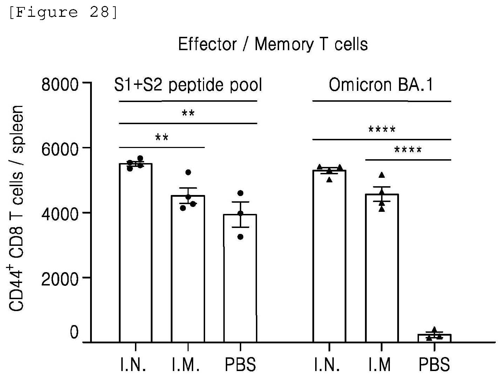

In addition, in the group receiving intranasal administration of the VLP vaccine containing saRNA, neutralizing antibody titers against Omicron BA.1 and Wuhan MA were significantly increased compared with the control group and the group receiving intramuscular (I.M.) administration of the vaccine (FIG. 25). Secretion of INF-γ, which is associated with innate immunity, was also increased (FIG. 26), and expression of the INF-α and INF-β genes was significantly increased (FIG. 27). The number of splenic INF-γ⁺ CD4⁺ T cells was also increased compared with the control group (FIG. 28).

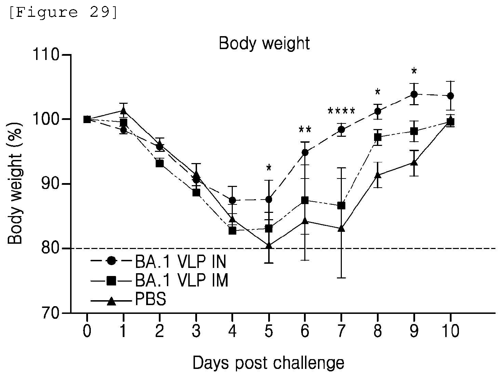

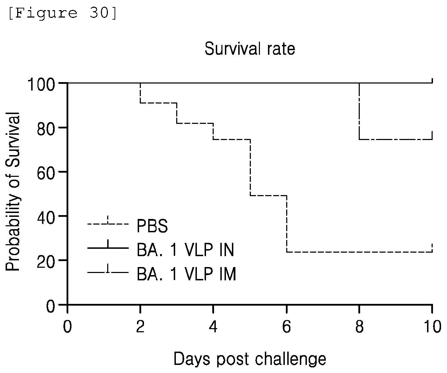

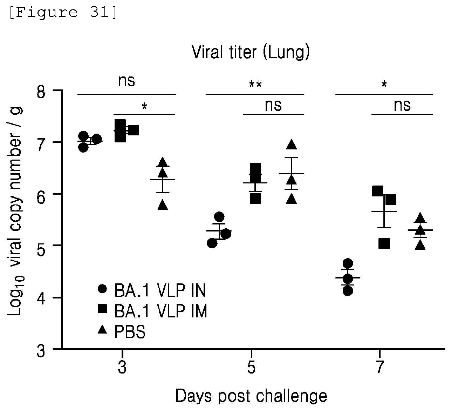

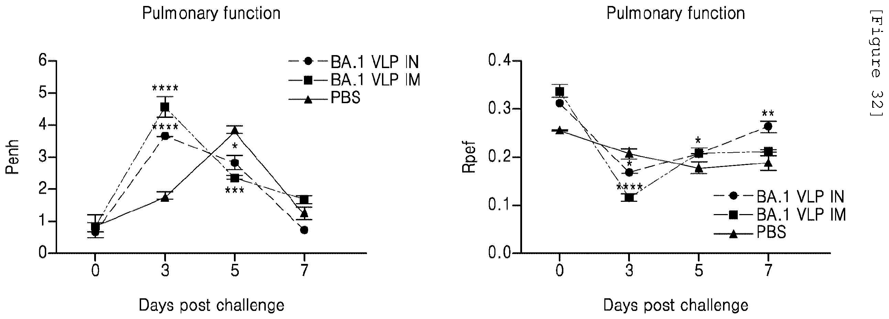

When the mice were challenged with Wuhan MA virus 28 days after administration of the VLP vaccine containing saRNA, the group receiving intranasal administration of the VLP vaccine containing saRNA showed faster recovery of body weight to a normal level than the control group and the intramuscular administration group (FIG. 29). The intranasal administration group also showed a significantly higher survival rate (FIG. 30), and the viral titer in the lungs of the mice rapidly decreased (FIG. 31). Consistent with these results, improvement in lung function in the mice was also significantly greater than in the control group (FIG. 32).

Taking these results together, when the VLP vaccine containing saRNA containing a sequence encoding the structural proteins of Omicron BA.1 was administered intranasally, i.e., when mucosal immunity was induced, mucosal immunity, T-cell immunity, innate immunity, and antibody-forming ability were markedly superior to those observed after intramuscular administration. In addition, even when the mice were challenged with virus 4 weeks later, adaptive immunity rapidly restored body weight and lung function toward normal levels, reduced lung viral titers, and improved survival.

These results indicated that the VLP vaccine can serve as an effective vaccine against SARS-CoV-2 virus and can be used as a vaccine suitable for mucosal administration.

### (3) Evaluation of Immunogenicity and Protective Efficacy (hACE2-Tg Mice)

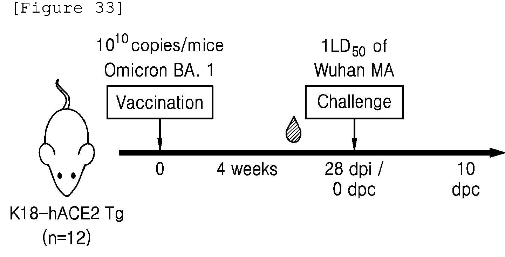

In the same manner as in (2) above, the VLP vaccine containing saRNA containing a sequence encoding the structural proteins of Omicron BA.1 was administered once intranasally (I.N.) at 10¹⁰ copies/mouse to hACE2-Tg mice expressing hACE2, a receptor used by SARS-CoV-2. Blood was collected from the mice, the mice were then challenged with 1 LD₅₀ of Wuhan MA virus, and blood samples were collected over 10 days to monitor the course of infection (FIG. 33).

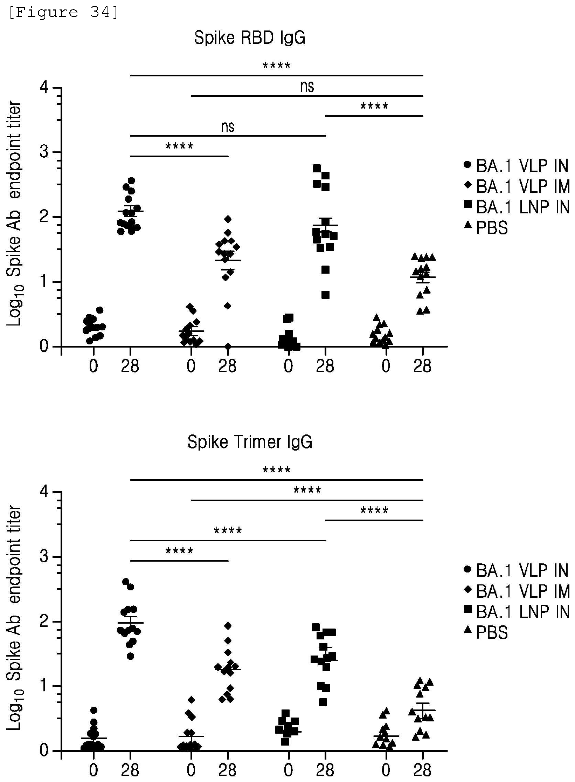

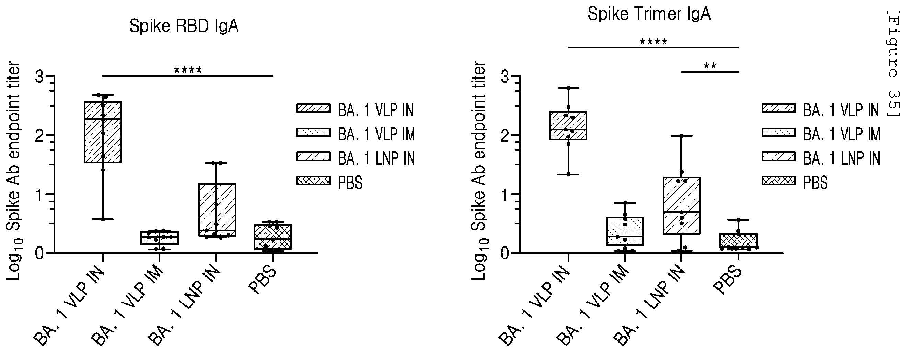

At 28 days after inoculation with the VLP vaccine containing saRNA, both α-spike RBD IgG and α-spike trimer IgG levels were significantly increased compared with the control group (PBS), the VLP vaccine intramuscular administration group, and the LNP vaccine intranasal administration group (FIG. 34). α-spike RBD IgA and α-spike trimer IgA, which indicate mucosal immunity, were also significantly increased compared with the control group (PBS), the VLP vaccine intramuscular administration group, and the LNP vaccine intranasal administration group. That is, for IgA, which is a mucosal immune antibody, the antibody titer was significantly higher only in the intranasal administration group. These results indicated that intranasal administration of the vaccine induced markedly enhanced antibody production (FIG. 35).

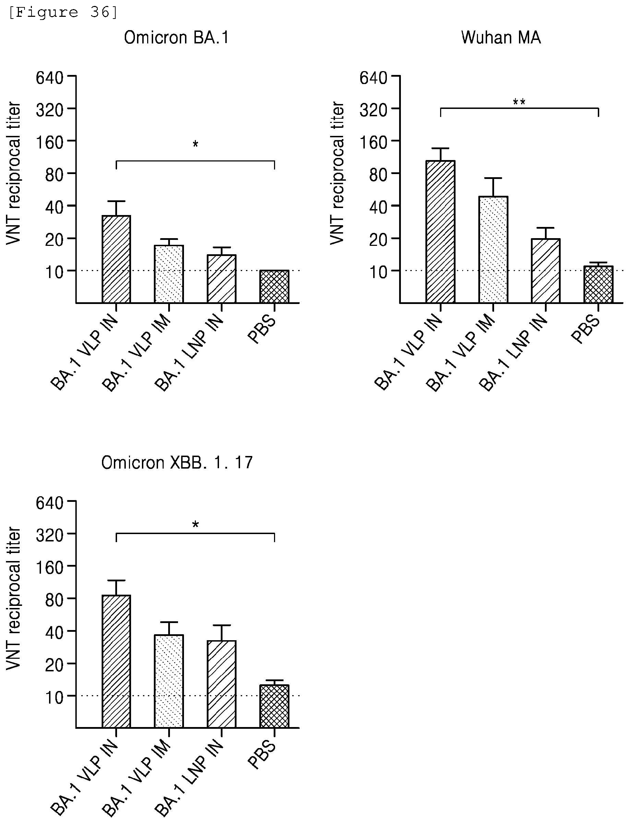

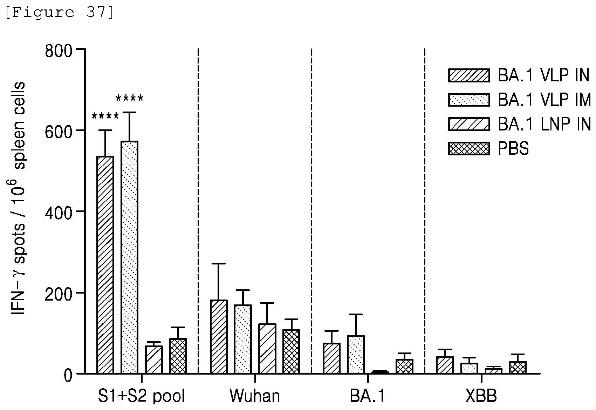

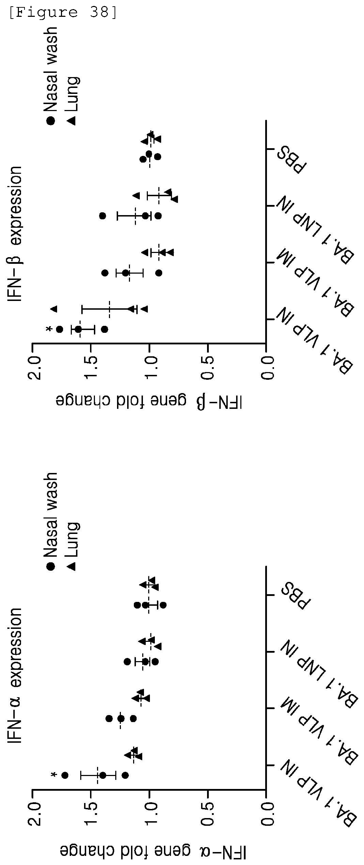

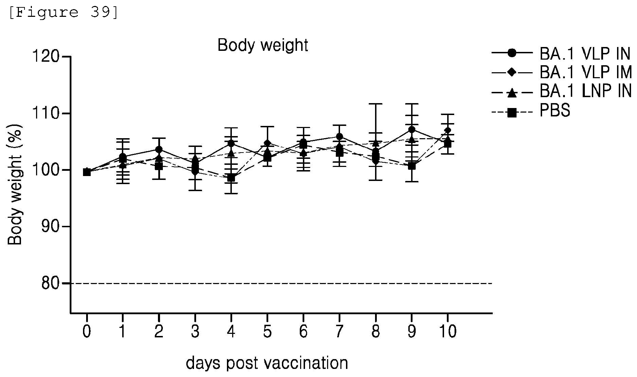

In addition, in the group receiving intranasal administration of the VLP vaccine containing saRNA, neutralizing antibody titers against Omicron BA.1, Wuhan MA, and Omicron XBB.1.17 were significantly increased compared with the control group (PBS), the VLP vaccine intramuscular administration group, and the LNP vaccine intranasal administration group (FIG. 36). Secretion of INF-γ, which is associated with innate immunity, was also increased (FIG. 37), and expression of the INF-α and INF-β genes was significantly increased (FIG. 38). Furthermore, the body weight of the mice in the group receiving intranasal administration of the VLP vaccine was maintained within a normal range without weight loss for up to 10 days after vaccine inoculation. These results indicated that the VLP vaccine safely induced a rapid immune response against the virus without apparent toxicity (FIG. 39).

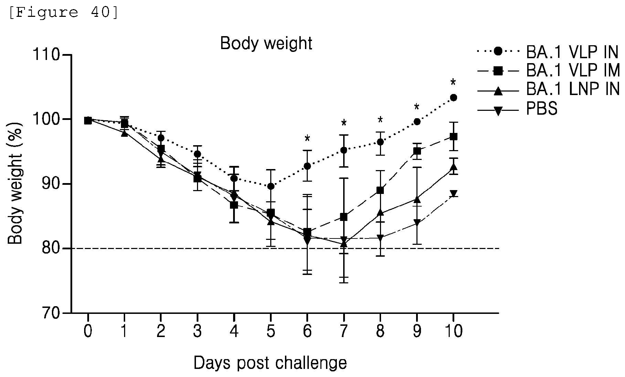

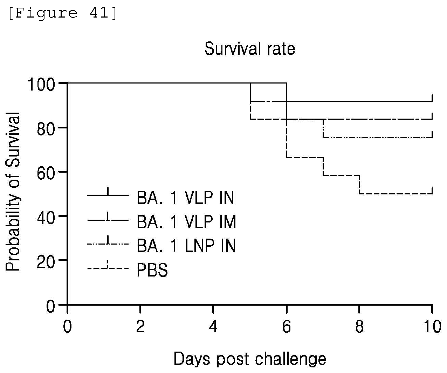

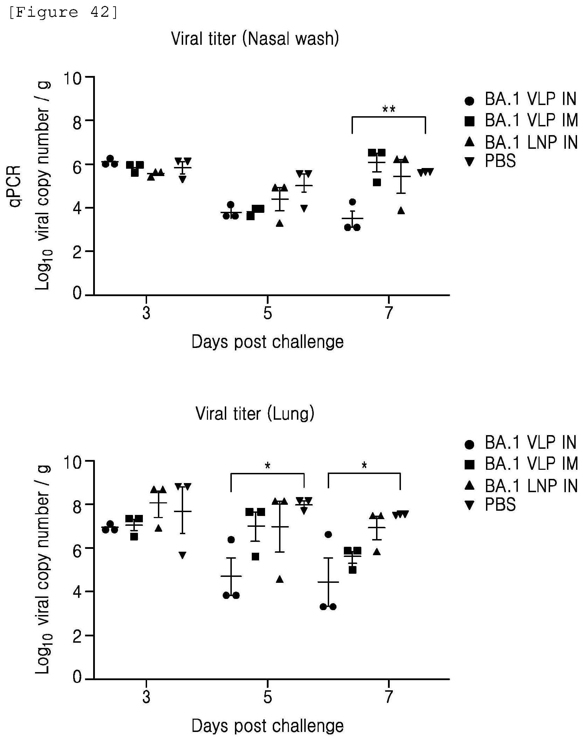

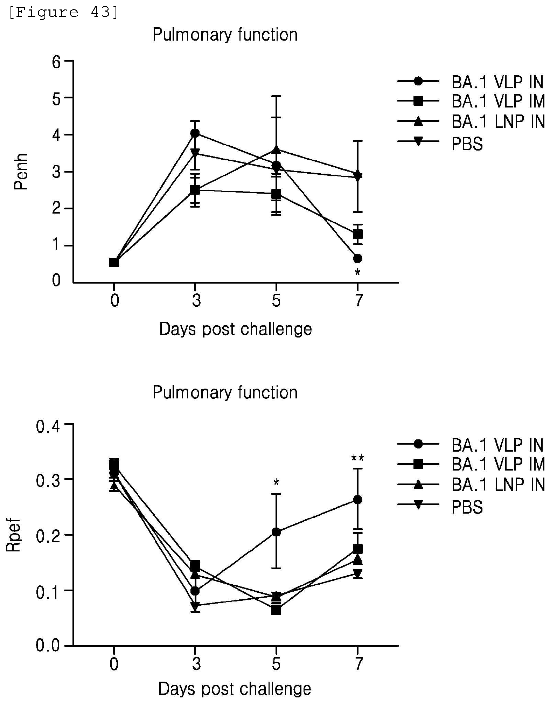

When the mice were challenged with Wuhan MA virus 28 days after administration of the VLP vaccine containing saRNA, the group receiving intranasal administration of the VLP vaccine containing saRNA showed faster recovery of body weight to a normal level than the control group (PBS), the VLP vaccine intramuscular administration group, and the LNP vaccine intranasal administration group (FIG. 40). The intranasal VLP vaccine administration group also showed a significantly higher survival rate (FIG. 41), and viral titers in the lungs and mucosal tissues of the mice rapidly decreased (FIG. 42). Consistent with these results, improvement in lung function in the mice was also significantly greater than in the control group (PBS), the VLP vaccine intramuscular administration group, and the LNP vaccine intranasal administration group (FIG. 43).

Taking these results together, when the VLP vaccine containing saRNA containing a sequence encoding the structural proteins of Omicron BA.1 was administered intranasally, i.e., when mucosal immunity was induced, mucosal immunity, T-cell immunity, innate immunity, and antibody-forming ability were markedly superior to those observed after intramuscular administration and intranasal administration of the LNP vaccine. In addition, even when the mice were challenged with virus 4 weeks later, adaptive immunity rapidly restored body weight and lung function toward normal levels, reduced lung viral titers, and improved survival.

Accordingly, these results indicated that the VLP vaccine can serve as a more effective vaccine against SARS-CoV-2 virus than a conventional LNP-type vaccine and can be used as a vaccine capable of inducing mucosal immunity through administration via the mucosal route, which corresponds to the route of SARS-CoV-2 infection.

### <Example 5> Use of the Vector System for Preparing VLPs Containing saRNA

It was examined whether a vector used to construct a vector system for preparing VLPs containing saRNA could be used after insertion of a foreign gene other than sequences encoding the structural proteins of Omicron BA.1, whether an additional antigen could be co-expressed, and whether the system could be applied to chimeric VLPs.

### (1) Evaluation of the Capability of the Vector System for VLP Preparation to Express a Foreign Gene

To determine whether a foreign gene other than a gene encoding the structural proteins of SARS-CoV-2 Omicron BA.1 could also be expressed, a replicon plasmid pSFV3-LacZ was prepared by subcloning a sequence encoding the LacZ protein into a pSFV3 backbone (containing sequences encoding nonstructural proteins NSP1 to NSP4 of Semliki Forest virus). RNA prepared from the linearized plasmid was then transfected into the BHK-21 cell line and the NP-293 cell line, and LacZ expression was examined on days 1 and 2 by betagalactosidase staining.

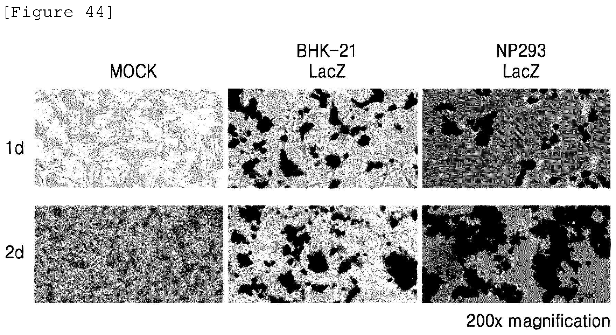

As shown in FIG. 44, LacZ protein expression was observed in both the BHK-21 cell line and the NP-293 cell line. Thus, these results indicated that the vector system for VLP preparation could be used by inserting, into the vector for VLP preparation, not only a gene encoding a protein derived from SARS-CoV-2 but also a foreign gene, for example, a gene encoding a protein derived from another virus.

### (2) Evaluation of Capability to Deliver an Additional Antigen in Addition to saRNA

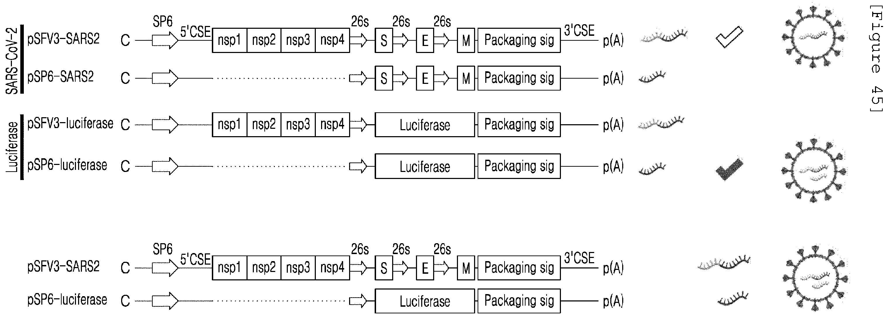

To evaluate the potential of the vector system as an additional antigen delivery system, a pSP6-luciferase vector was constructed by subcloning, into the pSP6-26s plasmid according to Preparation Example 1, a gene encoding luciferase, which served as an additional antigen, and a SARS-CoV-2 Omicron BA.1 packaging signal sequence. After this vector and pSFV3-SEM-PS9 (which is the same as pSFV3-SARS2) were linearized, transfection into the NP-293 cell line was performed, and expression of luciferase, the additional antigen, was examined (FIG. 45).

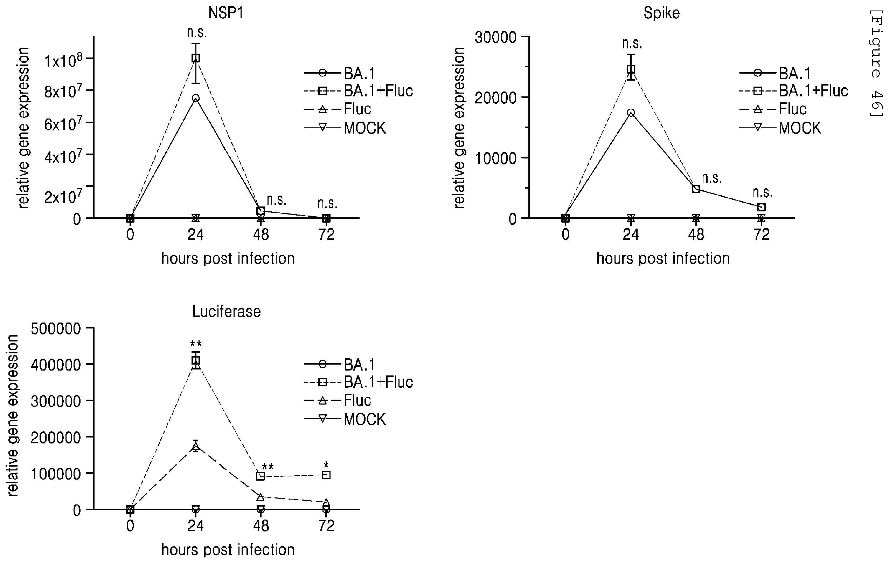

As shown in FIG. 46, expression of the NSP1-encoding gene, spikeencoding gene, and luciferase-encoding gene was examined, and all three genes showed high expression levels. These results indicated that the luciferase-encoding RNA co-transfected to express the additional antigen did not inhibit expression of the RNA for VLP preparation, i.e., did not compete with the RNA for VLP preparation. Rather, co-expression with the RNA for VLP preparation resulted in a higher luciferase expression level than transfection and expression of luciferase RNA alone.

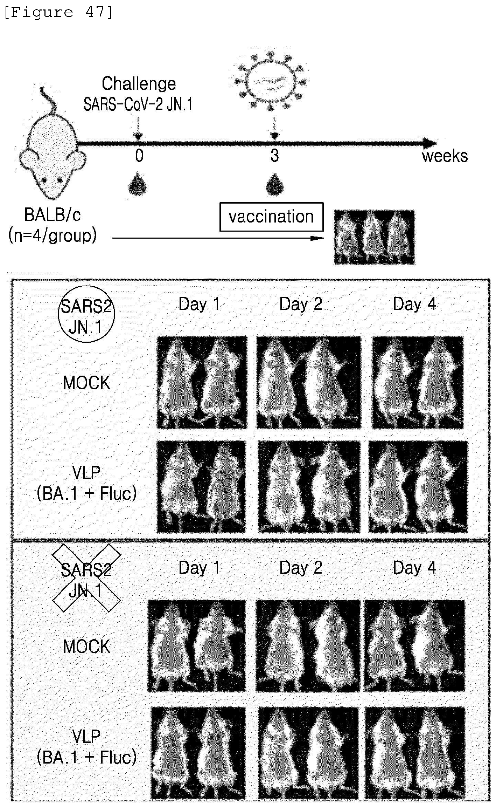

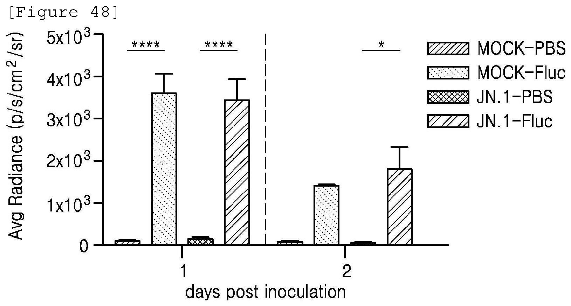

In addition, VLPs containing the additional antigen (luciferase) and saRNA (SARS-CoV-2 BA.1) were administered to BALB/c mice infected with SARS-CoV-2 JN.1 and to uninfected BALB/c mice, respectively (FIG. 47). As a result, expression of luciferase, the additional antigen, was observed in both SARS-CoV-2 JN.1-infected mice and uninfected mice (FIGS. 47 and 48). These results indicated that VLPs containing an additional antigen could be administered as a vaccine even to subjects with a history of viral infection or to subjects who had previously been vaccinated, that VLPs containing saRNA (SARS-CoV-2 BA.1) could function as an additional antigen delivery vehicle, and that the VLPs had markedly high antigen-delivery capability.

That is, when RNA prepared from a vector for VLP preparation and RNA prepared from a vector including a sequence encoding an additional antigen were co-transfected, RdRp expressed from the RNA for VLP preparation recognized the 26s sequence in the RNA sequence generated from the vector including the sequence encoding the additional antigen. As a result, expression of the additional antigen was significantly increased without interfering with expression of the saRNA. These results confirmed that the vector system for VLP preparation could also be used as a delivery vehicle for an additional antigen.

### (3) Evaluation of Capability to Prepare Chimeric VLPs

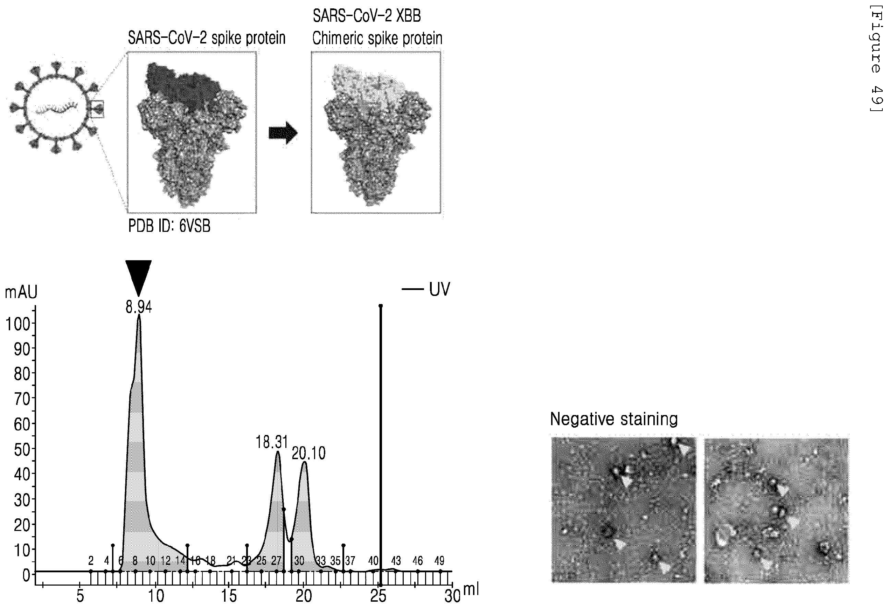

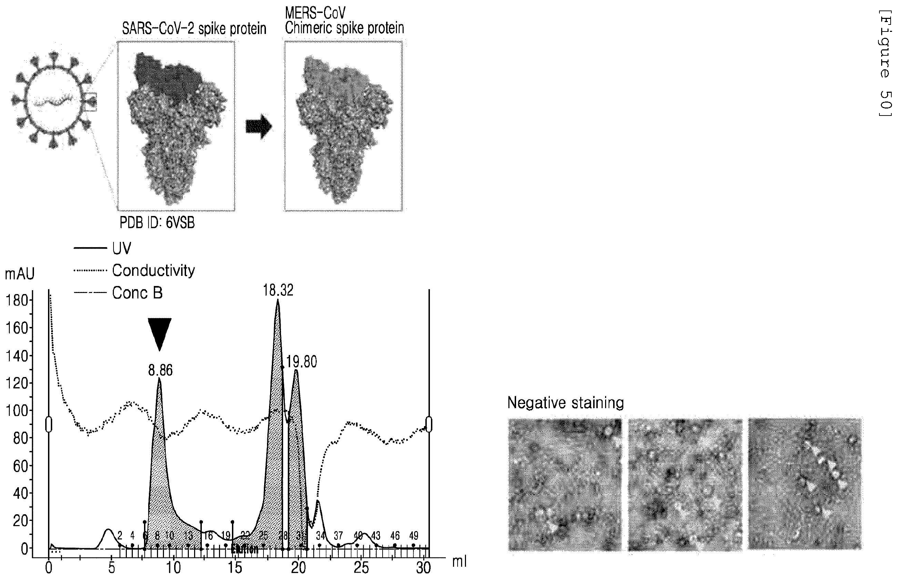

As shown in FIGS. 49 and 50, to use the vector system as a template applicable to vaccines against other viruses, it was examined whether chimeric VLPs could be prepared by altering the receptor-binding domain (RBD) in VLPs prepared using the vector system.

Specifically, when the receptor-binding domain of the spike protein of the existing SARS-CoV-2 Omicron BA.1 was changed to Omicron XBB.1 (hereinafter, XBB.1 chimeric VLP; FIG. 49) or MERS-CoV (hereinafter, MERS-CoV chimeric VLP; FIG. 50), VLPs containing the respective modified spike proteins were stably formed.

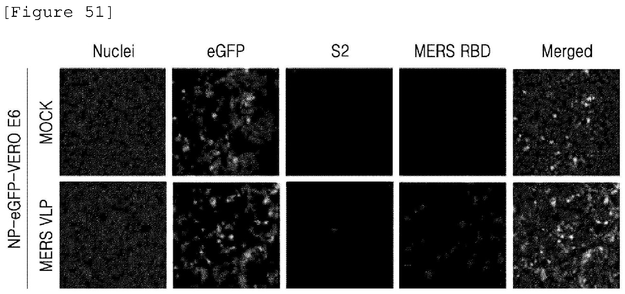

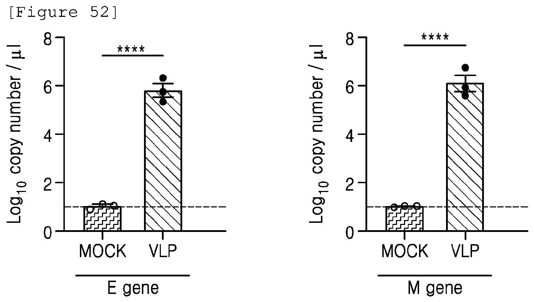

In addition, RNA generated from a vector for preparing the MERS-CoV chimeric VLP was transfected into the Vero E6 cell line, which is a large-scale VLP-producing cell line expressing the nucleocapsid protein. As a result, expression of the MERS-CoV RBD was detected (FIG. 51). Expression of genes encoding the envelope and membrane proteins of the MERS-CoV chimeric VLP was also detected (FIG. 52), indicating that chimeric VLPs could also be stably produced using the large-scale VLP-producing cell line.

That is, even when a portion of the vector system including sequences encoding viral structural proteins was replaced with sequences encoding structural proteins of another variant or another virus, VLPs were stably prepared and could also be produced on a large scale. These results confirmed that the vector system is useful as a vaccine template for various viruses.

### (4) Evaluation of Immunogenicity and Protective Efficacy of Chimeric VLPs

### (4)-1 Evaluation of Immunogenicity and Protective Efficacy (XBB.1 Chimeric VLP, BALB/c Mice)

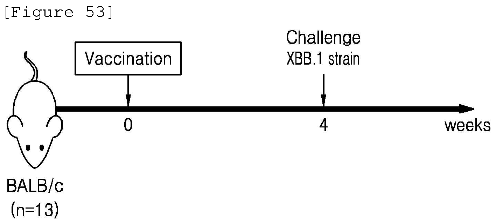

To evaluate immunogenicity and protective efficacy in mice following administration of the XBB.1 chimeric VLP, the XBB.1 chimeric VLP vaccine was intranasally (I.N.) administered to BALB/c mice, and blood was collected from the mice. The mice were then challenged with Omicron XBB.1 virus, and blood was collected for 10 days to monitor the course of infection (FIG. 53).

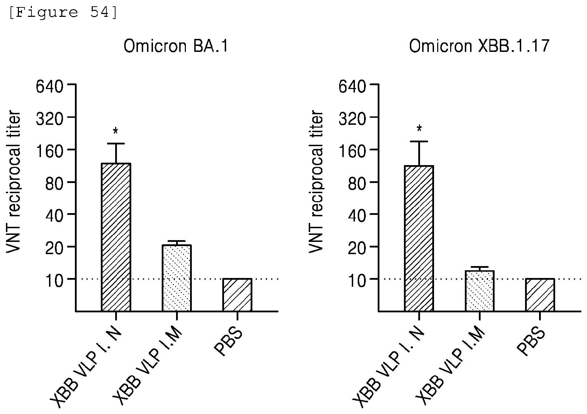

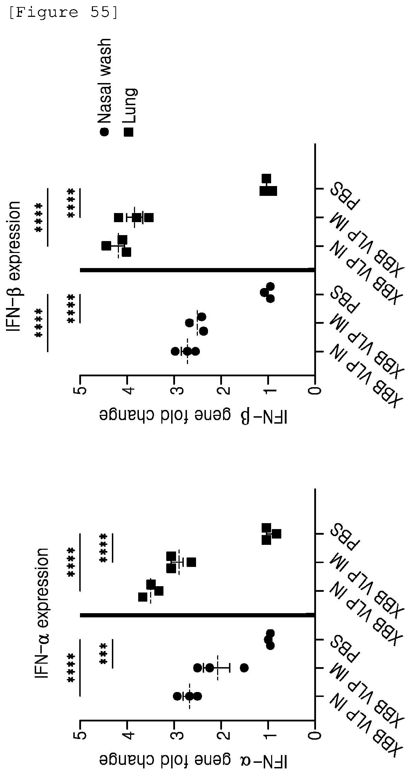

At 28 days after inoculation with the XBB.1 chimeric VLP vaccine, neutralizing antibody titers against Omicron BA.1 and Omicron XBB.1 were significantly increased in the group receiving intranasal administration of the XBB.1 chimeric VLP vaccine compared with the control group and the group receiving intramuscular (I.M.) administration of the vaccine (FIG. 54). In addition, expression of the INF-α and INF-β genes was significantly increased in the group receiving intranasal administration of the XBB.1 chimeric VLP vaccine compared with the control group (FIG. 55).

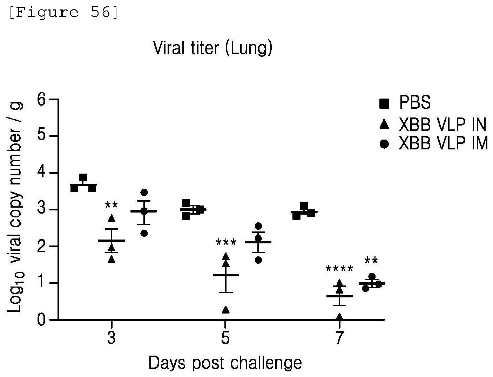

In addition, when the mice were challenged with Omicron XBB.1 virus 28 days after administration of the XBB.1 chimeric VLP vaccine, the viral titer in the lungs of the mice rapidly decreased in the group receiving intranasal administration of the XBB.1 chimeric VLP vaccine compared with the control group and the intramuscular vaccine administration group (FIG. 56).

Taking these results together, when the XBB.1 chimeric VLP vaccine was intranasally administered, i.e., when mucosal immunity was induced, the immune response was markedly superior to that in the control group (PBS) and the intramuscular administration group. In addition, even after viral challenge 4 weeks later, lung viral titers in the mice were rapidly reduced toward normal levels through adaptive immunity.

These results indicated that the chimeric VLP vaccine can serve as an effective vaccine against SARS-CoV-2 virus and can be used as a vaccine suitable for mucosal administration.

### (4)-2 Evaluation of Immunogenicity and Protective Efficacy (XBB.1 Chimeric VLP, hACE2-Tg Mice)

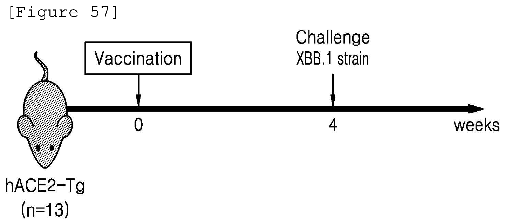

To evaluate immunogenicity and protective efficacy in mice following administration of the XBB.1 chimeric VLP, the XBB.1 chimeric VLP vaccine was administered intranasally (I.N.) to hACE2-Tg mice, and blood was collected from the mice. The mice were then challenged with Omicron XBB.1 virus, and blood samples were collected over 10 days to monitor the course of infection (FIG. 57).

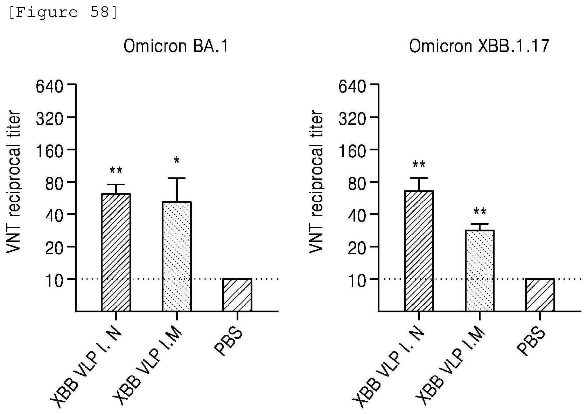

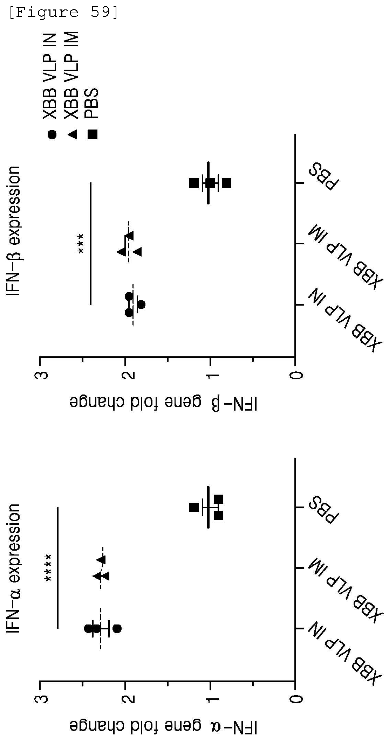

Consistent with the experimental results in BALB/c mice, at 28 days after inoculation with the XBB.1 chimeric VLP vaccine, neutralizing antibody titers against Omicron BA.1 and Omicron XBB.1 were increased in the group receiving intranasal administration of the XBB.1 chimeric VLP vaccine compared with the control group and the group receiving intramuscular (I.M.) administration of the vaccine (FIG. 58). In addition, expression of the INF-α and INF-β genes was significantly increased in the group receiving intranasal administration of the XBB.1 chimeric VLP vaccine compared with the control group (FIG. 59).

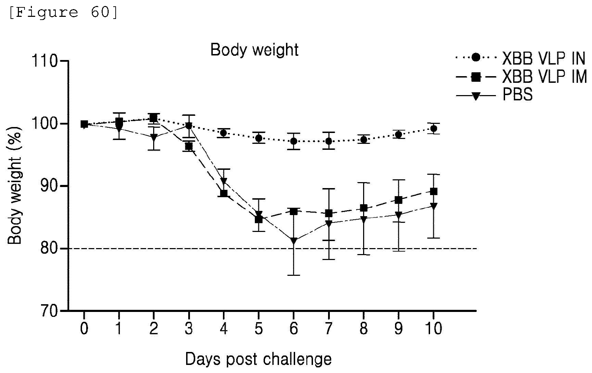

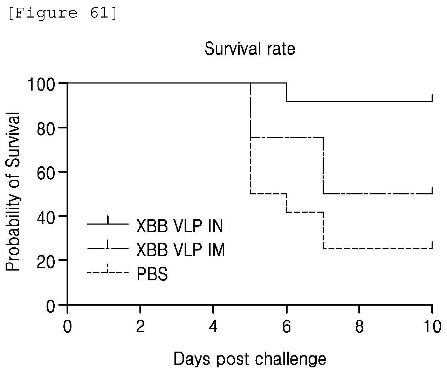

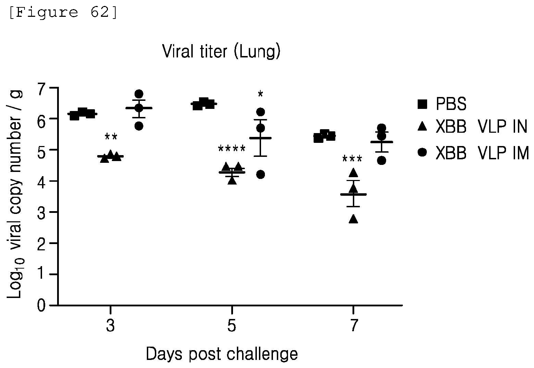

In addition, when the mice were challenged with Omicron XBB.1 virus 28 days after administration of the XBB.1 chimeric VLP vaccine, the group receiving intranasal administration of the XBB.1 chimeric VLP vaccine maintained a significantly higher survival rate and showed no decrease in body weight compared with the control group and the intramuscular vaccine administration group (FIGS. 60 and 61). Furthermore, the viral titer in the lungs of the mice also rapidly decreased (FIG. 62).

Taking these results together, when the XBB.1 chimeric VLP vaccine was administered intranasally, i.e., when mucosal immunity was induced, the immune response was markedly superior to that in the control group (PBS) and the intramuscular administration group. In addition, even after viral challenge 4 weeks later, lung viral titers were rapidly reduced toward normal levels through adaptive immunity, and a high survival rate was maintained through the superior immune response.

These results indicated that the chimeric VLP vaccine can serve as an effective vaccine against SARS-CoV-2 virus and can be used as a vaccine suitable for mucosal administration.

### (4)-3 Evaluation of Cell Infectivity, Immunogenicity, and Protective Efficacy (MERS-CoV Chimeric VLPs)

To determine whether the chimeric VLPs had cell infectivity, the VERO E6 cell line was infected with MERS-CoV chimeric VLPs, and expression of the MERS-CoV RBD protein was examined.

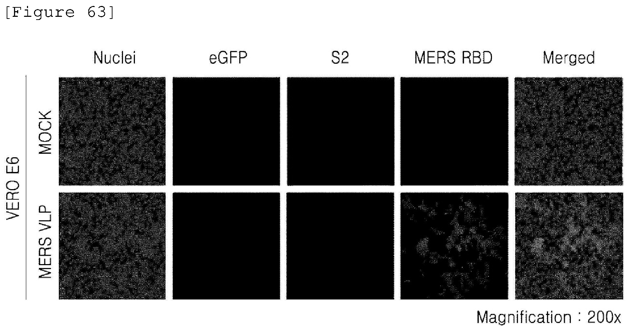

As shown in FIG. 63, when the VERO E6 cell line was infected with MERS-CoV chimeric VLPs, expression of the MERS-CoV RBD was detected. These results indicated that the MERS-CoV chimeric VLPs had cell infectivity.

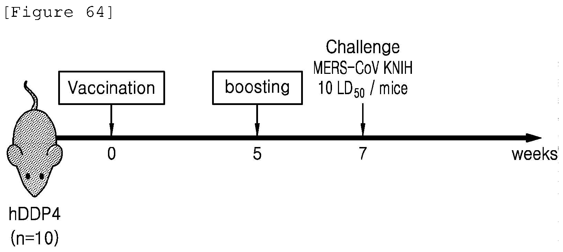

In addition, to evaluate immunogenicity and protective efficacy in mice following administration of the MERS-CoV chimeric VLPs, the MERS-CoV chimeric VLP vaccine was administered intranasally (I.N.) to hDPP4 mice, and blood was collected from the mice. The mice were then challenged with MERS-CoV virus, and blood samples were collected over 10 days to monitor the course of infection (FIG. 64).

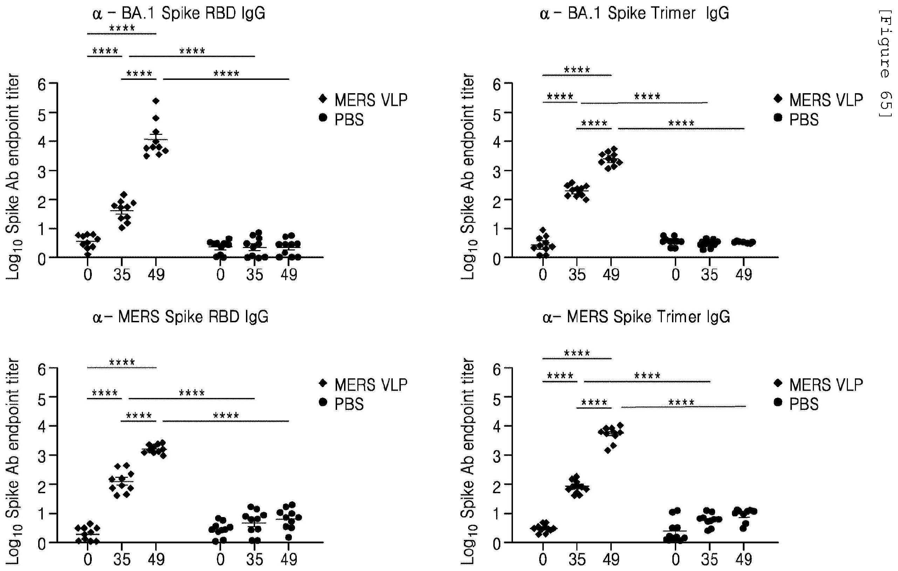

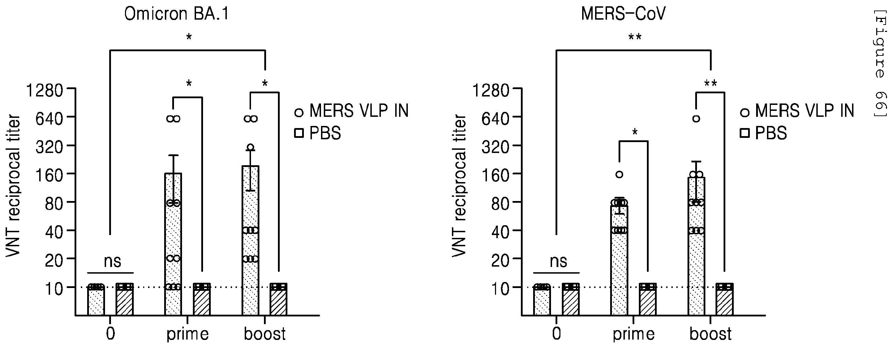

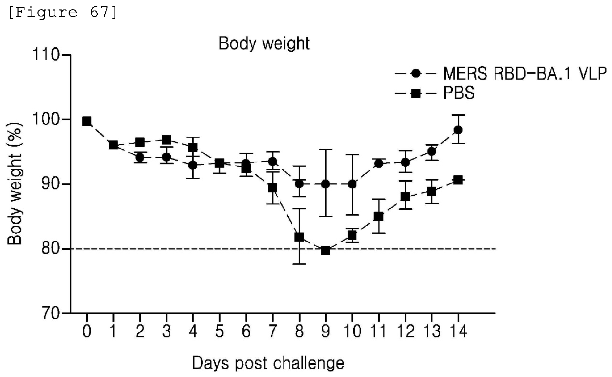

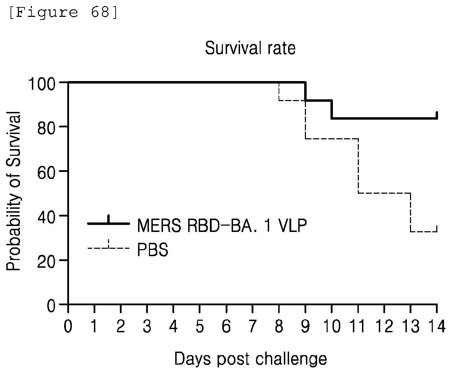

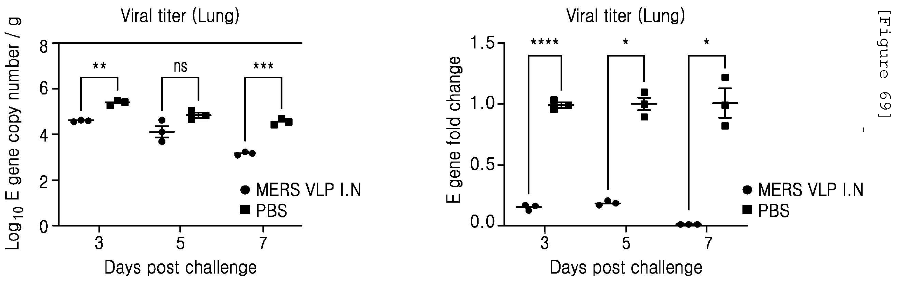

After inoculation with the MERS-CoV chimeric VLPs, levels of α-BA.1 spike RBD IgG, α-MERS spike RBD IgG, α-BA.1 spike trimer IgG, and α-MERS spike trimer IgG were all significantly increased compared with the control group (PBS) (FIG. 65). In addition, in the group receiving intranasal administration of the MERS-CoV chimeric VLP vaccine, neutralizing antibody titers against Omicron BA.1 and MERS-CoV were significantly increased compared with the control group (FIG. 66). When the mice were challenged with MERS-CoV after administration of the MERS-CoV chimeric VLP vaccine, the group receiving intranasal administration of the MERS-CoV chimeric VLP vaccine maintained a significantly higher survival rate and showed rapid recovery of body weight to a normal range compared with the control group (FIGS. 67 and 68). Furthermore, the viral titer in the lungs of the mice also rapidly decreased (FIG. 69).

Taking these results together, when the MERS-CoV chimeric VLP vaccine was administered intranasally, i.e., when mucosal immunity was induced, a markedly superior immune response was observed. In addition, even after viral challenge, lung viral titers were rapidly reduced toward normal levels through an adaptive immune response, and a high survival rate was maintained through the superior immune response.

These results indicated that the chimeric VLP vaccine can serve as an effective vaccine against coronaviruses and can be used as a vaccine suitable for mucosal administration.

## Claims

1. A vector comprising a gene encoding a nonstructural protein of an alphavirus and a gene encoding a target protein.

2. The vector of claim 1, wherein the alphavirus is one or more selected from the group consisting of Aura virus, Babanki virus, Barmah Forest virus, Bebaru virus, Cabassou virus, Chikungunya virus, Eastern equine encephalitis virus, Everglades virus, Fort Morgan virus, Getah virus, Highlands J virus, Kyzylagach virus, Mayaro virus, Me Tri virus, Middelburg virus, Mosso das Pedras virus, Mucambo virus, Ndumu virus, O'nyong-nyong virus, Pixuna virus, Rio Negro virus, Ross River virus, Salmon pancreas disease virus, Semliki Forest virus (SFV), Southern elephant seal virus, Tonate virus, Trocara virus, Una virus, Western equine encephalitis virus, and Whataroa virus.

3. The vector of claim 1, wherein the gene encoding the nonstructural protein of the alphavirus is one or more genes selected from the group consisting of a gene encoding NSP1 protein, a gene encoding NSP2 protein, a gene encoding NSP3 protein, and a gene encoding NSP4 protein.

4. The vector of claim 1, wherein the gene encoding the nonstructural protein of the alphavirus comprises a gene encoding RNA-dependent RNA polymerase (RdRp).

5. The vector of claim 1, wherein the target protein is a protein derived from a target virus or a fragment of the protein.

6. The vector of claim 5, wherein the gene encoding the protein derived from the target virus or a fragment of the protein is one or more selected from the group consisting of a gene encoding a spike protein derived from the target virus or a fragment of the spike protein, a gene encoding an envelope protein derived from the target virus or a fragment of the envelope protein, a gene encoding a membrane protein derived from the target virus or a fragment of the membrane protein, a gene encoding a packaging-signal-related protein derived from the target virus or a fragment of the packaging-signal-related protein, and a gene encoding a nucleocapsid protein derived from the target virus or a fragment of the nucleocapsid protein.

7. The vector of claim 5, wherein the target virus is a respiratory disease-causing virus.

8. The vector of claim 7, wherein the respiratory disease-causing virus is one or more selected from the group consisting of a coronavirus, a respiratory syncytial virus, an adenovirus, an influenza virus, a rhinovirus, an enterovirus, a bocavirus, a parainfluenza virus, a metapneumovirus, an Epstein-Barr virus, a cytomegalovirus, a herpes simplex virus, a measles virus, and a varicella-zoster virus.

9. A method of preparing a cell for producing virus-like particles (VLPs), the method comprising introducing RNA expressed from the vector of claim 1 into a cell.

10. The method of preparing a cell of claim 9, wherein introducing the RNA into the cell comprises co-introducing another RNA expressed from a vector comprising: a gene encoding a packaging-signal-related protein derived from a target virus; a polynucleotide derived from the alphavirus; and a gene encoding a second target protein.

11. The method of preparing a cell of claim 9, wherein the RNA comprises RNA encoding an RNA-dependent RNA polymerase of the alphavirus and RNA encoding the target protein.

12. The method of preparing a cell of claim 11, wherein the RNA encoding the target protein is one or more selected from the group consisting of RNA encoding a spike protein derived from a target virus or a fragment of the spike protein, RNA encoding an envelope protein derived from the target virus or a fragment of the envelope protein, RNA encoding a membrane protein derived from the target virus or a fragment of the membrane protein, and RNA encoding a packaging-signal-related protein derived from the target virus or a fragment of the packaging-signal-related protein; and
wherein the cell is genetically engineered to express a nucleocapsid protein derived from the target virus or a fragment of the nucleocapsid protein.

13. The method of preparing a cell of claim 9, further comprising linearizing the vector prior to the introduction of the RNA into the cell.

14. The method of preparing a cell of claim 9, wherein the cell is a microbial cell, an animal cell, or a plant cell.

15. A cell for producing virus-like particles, prepared by the method of claim 9.

16. A method of preparing virus-like particles, comprising culturing the cell of claim 15.

17. Virus-like particles prepared by the method of claim 16.

18. The virus-like particles of claim 17, wherein the target protein is a combination of a spike protein derived from a target virus or a fragment of the spike protein, an envelope protein derived from the target virus or a fragment of the envelope protein, and a membrane protein derived from the target virus or a fragment of the membrane protein.

19. The virus-like particles of claim 18, wherein the RNA is packaged with the spike protein derived from the target virus or a fragment of the spike protein, the envelope protein derived from the target virus or a fragment of the envelope protein, and the membrane protein derived from the target virus or a fragment of the membrane protein.

20. The virus-like particles of claim 17, further comprising RNA expressed from a vector comprising: a gene encoding a packaging-signal-related protein derived from a target virus; a polynucleotide derived from the alphavirus; and a gene encoding a second target protein.

21. A vaccine composition for preventing or treating a respiratory disease, comprising the virus-like particles of claim 17.

22. The vaccine composition of claim 21, wherein the vaccine composition induces a mucosal immune response.

23. The vaccine composition of claim 22, wherein the mucosal immune response comprises production of antigen-specific IgA in a mucosa.
